(19) Europäisches Patentamt
European Patent Office
Office européen des brevets

(11) Publication number: **0 457 243 A1**

(12) # EUROPEAN PATENT APPLICATION

(21) Application number: **91107721.2**

(22) Date of filing: **13.05.91**

(51) Int. Cl.5: **C07D 451/04**, C07D 451/14, C07D 453/02, C07D 453/06, C07D 487/08, C07D 209/80, A61K 31/40, A61K 31/435, //(C07D487/08,209:00,209:00)

(30) Priority: **14.05.90 US 523090**

(43) Date of publication of application: **21.11.91 Bulletin 91/47**

(84) Designated Contracting States: **AT BE CH DE DK ES FR GB GR IT LI LU NL SE**

(71) Applicant: **SYNTEX (U.S.A.) INC.**
**3401 Hillview Avenue**
**Palo Alto California 94304(US)**

(72) Inventor: **Berger, Jacob**
**12135 Dawn Lane**
**Los Altos Hills, CA 94022(US)**
Inventor: **Clark, Robin D.**
**2025 Columbia Street**
**Palo Alto, CA 94306(US)**
Inventor: **Eglen, Richard M.**
**2091 Sunnyview Lane**
**Mountain View, CA 94040(US)**
Inventor: **Smith, William L.**
**1169 Sesame Drive**
**Sunnyvale, CA 94087(US)**
Inventor: **Weinhardt, Klaus K.**
**155 Jackson Street**
**San Francisco, CA 94111(US)**

(74) Representative: **Barz, Peter, Dr. et al**
**Patentanwälte Dipl.-Ing. G. Dannenberg Dr. P. Weinhold, Dr. D. Gudel Dipl.-Ing. S. Schubert, Dr. P. Barz Siegfriedstrasse 8**
**W-8000 München 40(DE)**

(54) **Novel tricyclic compounds.**

(57) Compounds of Formula I:

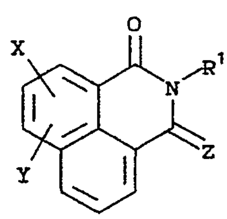

I

in which

Z is an oxo group or two hydrogens;

X and Y are independently selected from hydrogen, halogen, hydroxy, lower alkoxy, benzyloxy, lower alkyl, lower cycloalkyl, nitro, amino, amino-carbonyl, (lower alkyl)amino, di(lower alkyl)amino and (lower alkanoyl)-amino;

$R^1$ is selected from

EP 0 457 243 A1

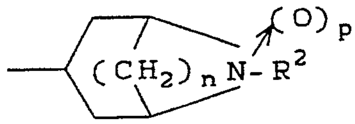

(a)

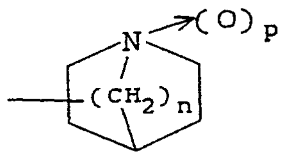

(b)

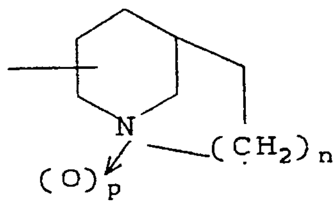

(c)

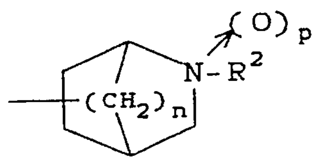

(d)

in which

p is 0 or 1;

n is 1, 2 or 3; and

$R^2$ is hydrogen, lower alkyl, $C_{3-8}$ cycloalkyl, $C_{3-8}$ cycloalkyl-$C_{1-2}$ alkyl, or a group $(CH_2)_tR^3$ where t is 1 or 2 and $R^3$ is thienyl, pyrrolyl or furyl optionally further substituted by one or two substituents selected from lower alkyl, lower alkoxy, trifluoromethyl or halogen, or is phenyl optionally substituted by one or two substituents selected from $C_{1-4}$ alkoxy, trifluoromethyl, halogen, nitro, carboxy, esterified carboxy, and $C_{1-4}$ alkyl optionally substituted by hydroxy, $C_{1-4}$ alkoxy, carboxy, esterified carboxy or in vivo hydrolyzable acyloxy; or their pharmaceutically acceptable salts, or an individual isomer or mixture of isomers thereof, the processes for the preparation thereof, as well as compositions containing them and their methods of use.

This invention relates to novel pharmaceutical tricyclic compounds, pharmaceutical compositions containing them and methods for their use and methods for preparing these compounds. In particular, it relates to azabicyclo-benzisoquinolines which interact with serotonin receptor subtypes. The invention also relates to novel intermediates useful in the synthesis of such compounds.

Compounds with highly selective actions on 5-HT (serotonin or 5-hydroxytryptamine) receptor subtypes show clear potential for therapeutic benefit and provide tools with which scientists can better understand the role of 5-HT in disease. A number of different 5-HT receptor subtypes have been identified. Some of these are designated as 5-$HT_1$, 5-$HT_2$ and 5-$HT_3$ receptors. Certain compounds having 5-$HT_3$ receptor mediating activity are useful for treating emesis, CNS disorders, cognitive performance disorders, drug dependency disorders, pain (e.g. migraine), cardiovascular disorders and gastrointestinal disorders. See, for example, an article entitled "Drugs Acting On 5-Hydroxytryptamine Receptors" appearing in The Lancet September 23, 1989.

Novel tricyclic compounds have now been discovered that are useful inter alia for treating a variety of conditions influenced by the 5HT receptors, in particular the 5-$HT_3$ receptor. The compounds of this invention are active at very low levels, particularly in the treatment of emesis but show also activity in the treatment of other disorders as shown below.

One aspect of this invention is a compound of Formula I:

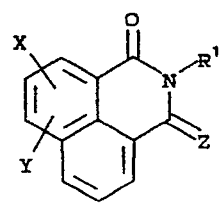

I

in which

Z is an oxo group or two hydrogens;

X and Y are independently selected from hydrogen, halogen, hydroxy, lower alkoxy, benzyloxy, lower alkyl, lower cycloalkyl, nitro, amino, amino-carbonyl, (lower alkyl)amino, di(lower alkyl)amino and (lower alkanoyl)amino;

$R^1$ is selected from

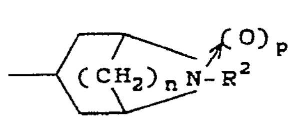

(a)

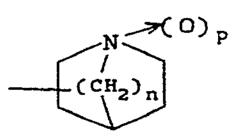

(b)

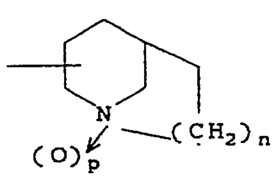

(c)

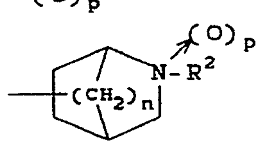

(d)

in which

p is 0 or 1;

n is 1, 2 or 3; and

$R^2$ is lower alkyl, $C_{3-8}$ cycloalkyl, $C_{3-8}$ cycloalkyl-$C_{1-2}$ alkyl, or a group $(CH_2)_tR^3$ where t is 1 or 2 and $R^3$ is thienyl, pyrrolyl or furyl optionally further substituted by one or two substituents selected from lower alkyl, lower alkoxy, trifluoromethyl or halogen, or is phenyl optionally substituted by one or two substituents selected from $C_{1-4}$ alkoxy, trifluoromethyl, halogen, nitro, carboxy, esterified carboxy, and $C_{1-4}$ alkyl optionally substituted by hydroxy, $C_{1-4}$ alkoxy, carboxy, esterified carboxy or in vivo hydrolyzable acyloxy; or

a pharmaceutically acceptable salt thereof, or an individual isomer or mixture of isomers thereof.

A second aspect of this invention is a pharmaceutical composition which contains a compound of Formula I, preferably in admixture with one or more suitable excipients.

A third aspect of this invention is a method for the treatment of emesis, nausea, migraine, gastrointestinal disorders, CNS disorders inclusive of obsessive compulsive behavior, stress- or not stress-related syndromes, panic disorders (experienced as apprehension, fear or terror) and agoraphobia, cardiovascular disorders such as arrhythmia, or pain, by administering a therapeutically effective amount of a compound of Formula I to a subject in need thereof.

A fourth aspect of this invention is the compounds of Formula IIIA and IIIB which are useful intermediates for preparing compounds of Formula I:

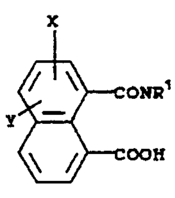

IIIA

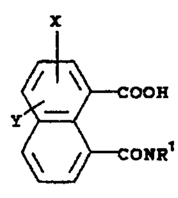

IIIB

wherein X, Y and $R^1$ are as defined for Formula I, and salts and reactive derivatives thereof.

A fifth aspect of this invention is a process for preparing compounds of Formula I and is set forth in the "Detailed Description of the Invention".

## Definitions

Unless otherwise stated, the following terms used in the specification and claims have the meanings given below:

"Alkyl" means a straight or branched saturated hydrocarbon radical having from one to the number of carbon atoms designated. For example, $C_{1-7}$ alkyl is alkyl having at least one but no more than seven carbon atoms, e.g. methyl, ethyl, *i*-propyl, *n*-propyl, *n*-butyl, pentyl, heptyl and the like.

"Cycloalkyl" means a cyclic hydrocarbon radical having from three to the number of carbon atoms designated. For example, $C_{3-8}$ cycloalkyl is cycloalkyl having at least three but no more than eight carbon atoms, e.g. cyclopropyl, cyclobutyl, cyclopentyl, cycloheptyl or cycloctyl.

"Lower alkyl" means an alkyl of one to six carbon atoms, i.e. $C_{1-6}$ alkyl.

"Lower alkoxy", "(lower alkyl)amino", "di(lower alkyl)amino", "(lower alkanoyl)amino", and similar terms mean alkoxy, alkylamino, dialkylamino, alkanoylamino, etc. in which the or each alkyl or alkanoyl radical contains from one to six carbon atoms.

"Halogen" means fluorine, chlorine, bromine, or iodine. Preferred halogens are chlorine and bromine.

"Pharmaceutically acceptable" means that which is useful in preparing a pharmaceutical composition that is generally safe and non-toxic and includes that which is acceptable for veterinary use as well as human pharmaceutical use.

"Pharmaceutically acceptable salts" means salts which possess the desired pharmacological activity and which are neither biologically nor otherwise undesirable. Such salts include acid addition salts formed with inorganic acids such as hydrochloric acid, hydrobromic acid, sulfuric acid, nitric acid, phosphoric acid, and the like; or with organic acids such as acetic acid, propionic acid, hexanoic acid, heptanoic acid, cyclopentanepropionic acid, glycolic acid, pyruvic acid, lactic acid, malonic acid, succinic acid, malic acid, maleic acid, fumaric acid, tartaric acid, citric acid, benzoic acid, o-(4-hydroxy-benzoyl)benzoic acid,

cinnamic acid, mandelic acid, methanesulfonic acid, ethanesulfonic acid, 1,2-ethanedisulfonic acid, 2-hydroxyethanesulfonic acid, benzenesulfonic acid, p-chlorobenzenesulfonic acid, 2-naphthalenesulfonic acid, *p*-toluenesulfonic acid, camphorsulfonic acid, 4-methyl-bicyclo[2.2.2]oct-2-ene-1-carboxylic acid, glucoheptonic acid, 4,4'-methylenebis(3-hydroxy-2-naphthoic) acid, 3-phenylpropionic acid, trimethyl-acetic acid, trifluoroacetic acid, tertiary butylacetic acid, lauryl sulfuric acid, gluconic acid, glutamic acid, hydroynaphthoic acid, salicylic acid, stearic acid, muconic acid, and the like. Preferred pharmaceutically acceptable salts are those formed with hydrochloric acid, maleic acid or hydrogen iodide.

The compounds of Formula I with an X or Y hydroxy group are capable of forming salts with inorganic or organic bases. Preferred pharmaceutically acceptable bases include sodium hydroxide, sodium carbonate, potassium hydroxide, aluminum hydroxide, calcium hydroxide and organic bases such as diethanolamine, tromethamine, N-methylglucamine, ethanolamine, triethanolamine and others.

"*N*-oxide derivative" of a compound means the form of a compound of Formula I wherein the nitrogen of the $R^1$ moiety of Formula I is in the oxidized state, i.e. where $p = 1$ in the formulae for $R^1$, e.g.

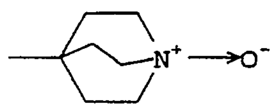

"Reactive Derivative" of a compound means the chemically activated form of a compound. The activated status facilitates the conversion of one compound into another compound. The term may refer to a compound of Formula I which can be converted to another compound of Formula I or may refer to an activated form of a reagent used in the preparation of a compound of Formula I.

Examples of reactive derivatives of a reagent of Formula IIIA or IIIB are mixed anhydrides of these compounds formed with lower alkanoic acids which facilitate the condensation to the compounds of Formula I which is described in more detail below. Other reactive derivatives will be disclosed in this specification in the description of the processes that are useful for the preparation of the compounds of Formula I and their conversion to other compounds of Formula I.

"Animal" includes humans, non-human mammals (such as dogs, cats, rabbits, cattle, horses, sheep, goats, swine, and deer) and non-mammals such as birds and the like.

"Disease" specifically includes any unhealthy condition of an animal or part thereof and may be caused by, or incident to, medical or veterinary therapy applied to that animal, i.e. the "side effects" of such therapy. Thus, "disease" here includes the nausea and emesis caused by therapy with agents having emetogenic side effects, in particular by therapy for cancer, such as chemotherapy with cytotoxic agents and radiotherapy.

"Treatment" means any treatment of a disease in an animal and includes:

(1) preventing the disease from occurring in an animal which may be predisposed to the disease but does not yet experience or display symptoms of the disease,

(2) inhibiting the disease, i.e. arresting its development, or

(3) relieving the disease, i.e causing regression of the disease.

"Effective amount" for a disease means that amount which, when administered to an animal in need thereof, is sufficient to effect treatment, as defined above, for that disease.

Certain compounds of Formula I may exist as optical isomers. In the compounds of the invention, any isomer or mixture of isomers may be used and the claims are intended to cover the individual isomer and mixtures thereof, unless otherwise restricted. The invention includes all optical isomers of any asymmetrical compound of Formula I, as well as mixtures thereof.

"Isomerism" refers to compounds having the same atomic mass and atomic number but differing in one or more physical or chemical properties. Various types of isomers include the following:

"Stereoisomer" refers to a chemical compound having the same molecular weight, chemical composition, and constitution as another, but with the atoms grouped differently. That is, certain identical chemical moieties are at different orientations in space and, therefore, when pure, have the ability to rotate the plane of polarized light. However, some pure stereoisomers may have an optical rotation that is so slight that it is undetectable with present instrumentation.

"Optical isomer" describes one type of stereo isomerism which manifests itself by the rotation that the

isomer, either pure or in solution, imparts to the plane of polarized light. It is caused in many instances by the attachment of four different chemical atoms or groups to at least one of the carbon atoms in a molecule.

Stereoisomers or optical isomers that are mirror images of one another are termed "enantiomers" and may be said to be enantiomeric. Chiral groups that are mirror images of one another are termed enantiomeric groups.

Enantiomers whose absolute configurations are not known may be differentiated as dextrorotatory (prefix +) or laevorotatory (prefix -) depending on the direction in which, under specified experimental conditions, they rotate the plane of polarized light.

When equal amounts of enantiomeric molecules are present together, the product is termed racemic, independently of whether it is crystalline, liquid, or gaseous. A homogeneous solid phase composed of equimolar amounts of enantiomeric molecules is termed a racemic compound. A mixture of equimolar amounts of enantiomeric molecules present as separate solid phases is termed a racemic mixture. Any homogeneous phase containing equimolar amounts of enantiomeric molecules is termed a racemate.

"Diastereoisomer" refers to stereoisomers some or all of which are dissymmetric but which are not mirror images of each other. Diastereoisomers corresponding to a given structural formula must have at least two asymmetric atoms. A compound having two asymmetric atoms will usually exist in four diastereoisomeric forms, i.e. (-)-erythro, (+)-erythro, (-)-threo and (+)-threo.

The optically active compounds herein can be designated by a number of conventions; i.e., the *R*- and *S*-sequencing rules of Cahn and Prelog; erythro and threo isomers; D- and L-isomers; d- and l-isomers; and (+) and (-) isomers, which indicates the direction a plane of polarized light is rotated by the chemical structure, either pure or in solution. These conventions are well known in the art and are described in detail by E.L. Eliel in Stereochemistry of Carbon Compounds, published by McGraw Hill Book Company, Inc. of New York in 1962 and references cited therein. Thus, these isomers may be described as d-, l-, or a d,l-pair; or D-, L-, or a D,L-pair; or *R*-, *S*-, or an *R,S*-pair; depending upon the nomenclature system employed. In general, this application will use the (*R*), (*S*) and (*RS*) designation.

"Optional" or "optionally" means that the subsequently described event or circumstance may or may not occur, and that the description includes instances where said event or circumstance occurs and instances in which it does not. For example, "optionally substituted phenyl" means that the phenyl may or may not be substituted and that the description includes both unsubstituted phenyl and phenyl wherein there is substitution; "optionally followed by converting the free base to the acid addition salt" means that said conversion may or may not be carried out in order for the process described to fall within the invention, and the invention includes those processes wherein the free base is converted to the acid addition salt and those processes in which it is not.

Certain $R^1$ substituents are of particular interest for the compounds of this invention and are therefore defined specifically. In some cases the $R^1$ substituent will exhibit a chiral center at the ring carbon which is bonded to the isoquinoline nitrogen. It is to be understood that a straight line representing the covalent bond between the chiral carbon and the isoquinoline nitrogen is understood to represent either the *R* or *S* configuration, or a mixture (not necessarily racemic) thereof. In some instances compounds of Formula I will exist as the *endo* or *exo* form relative to the $R^1$ substituent. For purposes of the present application when referring to a named compound and *endo* or *exo* is not designated, it is to be understood that the reference is to both forms. These $R^1$ substituents of particular interest are as follows:

(1) subformula (b) where n is 2 and p is 0 having the specific formula

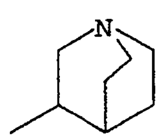

is referred to as 1-azabicyclo[2.2.2]oct-3-yl;

(2) subformula (b) where n is 2 and p is 0 having the specific formula

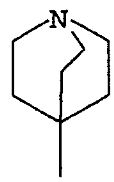

is referred to as 1-azabicyclo[2.2.2]oct-4-yl;
(3) subformula (a) where n is 3, p is 0 and $R^2$ is methyl having the specific formula

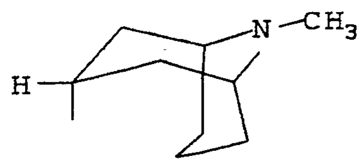

is referred to as *endo*-9-methyl-9-azabicyclo[3.3.1]non-3-yl;
(4) subformula (a) where n is 3, p is 0 and $R^2$ is methyl having the specific formula

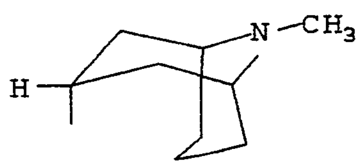

is referred to as *exo*-9-methyl-9-azabicyclo[3.3.1]non-3-yl;
(5) subformula (a) where n is 2, p is 0 and $R^2$ is methyl having the specific formula

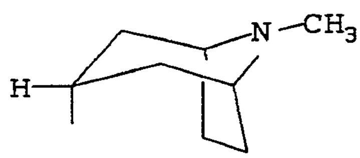

is referred to as *endo*-8-methyl-8-azabicyclo[3.2.1]oct-3-yl;
(6) subformula (a) where n is 2, p is 0 and $R^2$ is methyl having the specific formula

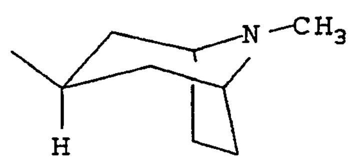

is referred to as *exo*-8-methyl-8-azabicyclo[3.2.1]oct-3-yl;
(7) subformula (c) wherein n is 2 and p is 0 having the specific formula

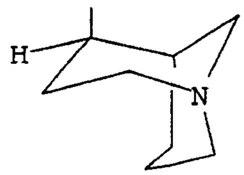

is referred to as *endo*-1-azabicyclo[3.3.1]non-4-yl; and

(8) subformula (c) wherein n is 2 and p is 0 having the specific formula

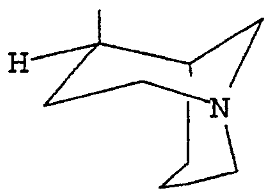

is referred to as *exo*-1-azabicyclo[3.3.1]non-4-yl.

The temperature conditions and reaction times provided in the specification and the example section apply to laboratory conditions, unless stated otherwise. At a larger or commercial scale reaction temperatures and times may vary.

The term "yield" when used herein refers to yield relative to the expected theoretical yield.

The compounds of Formula I are named in accordance with acceptable nomenclature rules generally consistent with "Chemical Abstracts". For example, the compound of Formula I wherein Z is the oxo group (a compound of Formula IA) and $R^1$ is 1-azabicyclo-[2.2.2]oct-3-yl

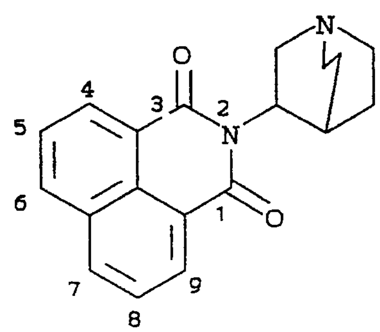

IA

is named 2-(1-azabicyclo[2.2.2.]oct-3-yl)-2,3-dihydro-1*H*-benz[*de*]isoquinoline-1,3-dione.

Although deviating from accepted rules of nomenclature, but to be consistent with the general Formula I, the compound of Formula I wherein Z stands for two hydrogens (a compound of Formula IB) and $R^1$ is 1-azabicyclo-[2.2.2]oct-3-yl

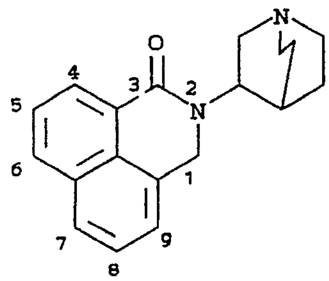

IB

is named 2-(1-azabicyclo[2.2.2.]oct-3-yl)-2,3-dihydro-1*H*-benz[*de*]isoquinoline-3-one.

Although deviating from accepted rules of nomenclature, but to be consistent with the general Formula I, the following Formula III wherein $R^1$ is 1-azabicyclo-[2.2.2]oct-3-yl

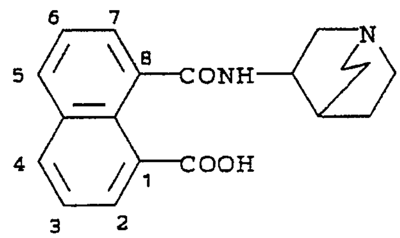

III

is named 8-(1-azabicyclo[2.2.2.]oct-3-yl)aminocarbonylnaphthalene-1-carboxylic acid.

Utility

The compounds of this invention, as defined by Formula I, exhibit pharmaceutical activity in particular at serotonin receptors. As such, these compounds are useful for treating a broad range of conditions in animals, particularly humans, in which the serotonin receptor plays a role. Examples of conditions that may be treated using the compounds of this invention include nausea and emesis, migraine, gastrointestinal disorders, central nervous system (CNS) disorders, such as obsessive compulsive behavior, stress- or non stress-related syndromes such as panic disorders, phobias such as agoraphobia, anxieties, cognitive performance disorders, drug dependency etc., cardiovascular disorders such as arrythmia and pain.

For purposes of this patent application, particularly the claims the term "emesis" will have a meaning that is broader than the normal, dictionary definition and includes not only vomiting, but also nausea and retching. Such a condition of emesis may be induced by or result from the administration of chemotherapeutic or cytotoxic agents or radiation in cancer treatment, or from exposure to radiation, surgical operations or anesthesia or motion sickness (caused by riding in a vehicle, airplane, vessel, etc.). Thus, the compounds of this invention can be referred to as anti-emetics and are particularly valuable for treating (especially preventing) emesis induced in cancer patients by treatments with cytotoxic or chemotherapeutic pharmaceutical agents or radiation. Such cytotoxic agents include platinum anti-cancer agents such as cisplatin (*cis*-diamminedichloroplatinum), as well as non-platinum anti-cancer drugs such as cyclophosphamide (cytoxin), vincristrine (leurocristine), procarbazine (*N*-(1-methylethyl)-4-[(2-methylhydrazino)methyl]benzamide), methotrexate, fluorouracil, mechlorethamine hydrochloride (2-chloro-*N*-(2-chloroethyl)-*N*-methylethanamine hydrochloride), doxorubicin, adriamycin, dactinomycin (actinomycin-D), cytarabine, carmustine, dacarbazine, and derivatives and prodrugs of these compounds, and others listed at page 1143 of the Journal of Clinical Oncology 1989; 7(*8*): 1143. The compounds of the invention may also be useful for treating post-operative nausea and vomiting and motion sickness.

The gastrointestinal disorders that are treatable using the compounds of this invention are those in

which the normal GI motility is altered as a result of serotonin receptor interaction (e.g. in the stomach, esophagus and both the large and small intestines). Examples of specific conditions that may be treated using the compounds of this invention include, but are not limited to, dyspepsia (including non-ulcer dyspepsia), gastric stasis, peptic ulcer, reflux esophagitis, flatulence, bile reflux gastritis, pseudo-obstruction syndrome, irritable colon syndrome (which may result in chronic constipation or diarrhea), diverticular disease, biliary dysmotility (which may result in sphincter of Oddi dysfunction and "sludge" or microscopic crystals in the gall bladder), gastroparesis (e.g. diabetic, postsurgical or idiopathic), irritable bowel syndrome and retarded gastric emptying. The compounds of the invention are also useful as short-term prokinetics to facilitate diagnostic radiology and intestinal intubation. In addition the compounds are useful for treating diarrhea, particularly diarrhea induced by cholera and carcinoid syndrome.

CNS disorders are usually associated with an abnormality in levels of neurotransmitters or their release. 5-HT receptors regulate dopaminergic, adrenergic and cholinergic neuronal activity, and serotonin receptors are present in those areas of the brain involving mood, emotion, reward and memory. Categories of treatable CNS disorders include cognitive disorders, psychoses, obsessive/compulsive and anxiety/depression behavior. Cognitive disorders include attentional or memory deficit, dementia states (including senile dementia of the Alzheimer's type and aging), cerebral vascular deficiency and Parkinson's disease. Psychoses that may be treated using the compounds of this invention include paranoia and schizophrenia. Representative, treatable anxiety/depressive states include anticipatory anxiety (e.g. prior to surgery, dental work, etc.), depression, mania, convulsions and anxiety caused by withdrawal from addictive substances such as nicotine, alcohol, diazepam, common narcotics, cocaine and other drugs of abuse, social phobias, simple phobias, post-traumatic stress disorders, and generalized anxiety disorders. Obsessive/compulsive behavior, e.g. that results from obesity, may be treated using the compounds of this invention.

Cardiovascular disorders that may be treated using a compound of this invention are those that are mediated by the presence of serotonin. Examples of such disorders include arrhythmias and hypertension.

The compounds of this invention may also be useful in the treatment of anxiety disorders other than directly stress-induced anxiety disorders, particularly panic disorders or attacks and various phobias including agoraphobia.

Pain that is treatable using a compound of this invention is that which is created by a mechanism that alters neuronal traffic. It is thought that the compounds of this invention prevent certain adverse nervous transmissions and/or prevent vasodilation and thus reduce the perceived level of pain. Examples of pain treatable using a compound of this invention include cluster headaches, migraines, trigeminal neuralgia and visceral pain (e.g. that caused by abnormal distension of hollow visceral organs).

To determine the serotonergic activity of compounds of this invention, one of ordinary skill may use the Rat Cerebral Cortex Binding Assay, a predictive *in vitro* assay which assesses the binding affinity of a compound for the $5\text{-}HT_3$ receptor. The method is described in Kilpatrick, G.J., Jones, B.J. and Tyers, M.B., Nature 1987; *330*: 24-31. The assay as adapted for testing compounds of the invention is set out in Example 7 of this application.

The von Bezold-Jarisch test for $5\text{-}HT_3$ antagonist activity in rats is an accepted test for determining $5\text{-}HT_3$ antagonist activity *in vivo* by measuring the von Bezold-Jarisch reflex in anesthetized rats. See, e.g., Butler, A., Hill, J.M., Ireland, S.J., Jordan, C.C., Tylers, M.B., Brit. J. Pharmacol. 1988; *94*: 397-412; Cohen, M.L., Bloomquist, W., Gidda, J.S., Lacefield, W., J. Pharmacol. Exp. Ther. 1989; *248*: 197-201; and Fozard, J.R., MDL 72222: Arch. Pharmacol. 1984; *326*: 36-44. The compounds of the invention exhibit activity in the von Bezold-Jarisch test. The details of the procedure (as modified for testing the compounds of the invention) and results are set out in Example 8 of this application.

The cisplatin-induced emesis test in ferrets is an accepted test for determining anti-emetic activity *in vivo*, see e.g. Costall, B., Domeney, A.M., Naylor, R.J., and Tattersall, F.D., Neuropharmacology 1986; *25(8)*: 959-961; and Miner, W.D. and Sanger G.J., Brit. J. Pharmacol. 1986; *88*: 497-499. A general description is set out in Example 9 of this application.

Anti-emetic properties in the control of emesis in dogs due to administration of platinum anti-cancer drugs are also determined by a modification of the method described by Smith, W.L., Alphin, R.S., Jackson, C.B., and Sancilio, L.F., J. Pharm. Pharmacol. 1989; *41*: 101-105; and Gylys, J.A., Res. Commun. Chem. Pathol. Pharmacol. 1979; *23(1)*: 61-68 as follows: cisplatin (*cis*-diamminedichloroplatinum) is administered at a dose of 3 mg/kg intravenously to non-fasted dogs (both sexes). Sixty minutes after cisplatin administration, either vehicle or the test drug in saline at a dose volume of 0.1 ml/kg and 1.0 mg/kg, respectively is administered intravenously. A control group of dogs is given the cisplatin followed by saline at 60 min, without test drug. The dogs are observed continuously for a period of 5 hrs counting the number of emetic episodes and comparing them to emetic episodes observed for the controls. Details of the

procedure are set out in Example 10 of this application.

The utility for treating gastrointestinal disorders is determined by assaying the gastrokinetic pharmacological activity using the method of Droppleman, D., Gregory, R., and Alphin, R.S., J. Pharmacol. Methods 1980; *4(3)*: 227-30 wherein the rate of emptying of a test meal in rats compared to controls was observed. The Droppleman et al. method is an accepted method for determining gastrointestinal activity *in vivo*. The compounds of the invention exhibit activity in the Droppleman et al. method, the detail of which is set out in Example 11.

The utility for treatment of a CNS disorder such as anxiety (anxiolytic activity) is determined by the art-recognized Crawley and Goodwin two-compartment exploratory model as described in Kilfoil, T., Michel, A., Montgomery, D., and Whiting, R.L., Neuropharmacology 1989; *28(9)*: 901-905. In brief, the method involves determining whether a compound reduces the natural anxiety of mice in brightly-lit areas. Compounds of the invention are active in this art ecognized test. An example is set forth in Example 12 of this application.

Anxiolytic activity during withdrawal from drugs of abuse is determined by the mouse light/dark withdrawal anxiety test. This procedure utilizes the exploratory model described above to test for anxiolytic activity after administration and subsequent abrupt cessation of diazepam, alcohol, cocaine or nicotine treatments. A detailed description is set for in Example 13 of this application. Compounds of Formula I are effective at reversing the drug withdrawal-induced anxiety in this test.

Cognition enhancing activity may be determined by the mouse habituation/cognitive enhancement test. See procedures described in Barnes, J.M., Costall, B., Kelly, M.E., Naylor, F.J., Onaivi, E.S., Tomkins, D.M. and Tyers, M.B. Br. J. Pharmacol. 98, 693P (1989). This procedure utilizes the exploratory model described above to test for improvements in the impaired cognitive performance of aged mice. A detailed description is set forth in Example 14 of this application. Compounds of Formula I enhance cognitive performance in this test.

All of the aforementioned citations to methods for determining activity of the compounds of this invention and other documents cited herein are incorporated herein by reference.

In summary, this invention relates to methods for treating animals exhibiting conditions in which the serotonin receptor plays a role, e.g. where the condition is chosen from emesis, migraine, a gastrointestinal disorder, a CNS disorder, a cardiovascular disorder and pain, which method comprises administering a therapeutically effective amount of a compound of formula I to such mammal. Additionally, the invention relates to methods for treating animals exhibiting conditions in which anxiety or phobia is involved, wherein said method comprises administering a therapeutically effective amount of compound of Formula I to such animal.The compounds are particularly valuable for treating humans.

A therapeutically effective amount of a compound is an amount that is efficacious in treating the condition, i.e. the disease. The exact amount administered may vary over a wide range depending on the degree of severity of the specific condition being treated, age of the subject, relative health of the subject and other factors. A therapeutically effective amount may vary from about 0.000001 mg (1 nanogram [ng]) per Kg body weight per day to about 10.0 mg/Kg body weight per day. Preferably the amount will be about 10 ng/Kg/day to about 1.0 mg/Kg/day, especially for anti-emetic purposes. Thus, for a 70 Kg human, a therapeutically effective amount may be from about 70 ng/day to 700 mg/day, preferably about 700 ng/day to about 70 mg/day.

## Administration and Pharmaceutical Compositions

The compounds of this invention may be administered via any of the usual and acceptable modes known in the art, either singly or in combination with another compound of this invention or with another therapeutic agent. Generally a compound of this invention is administered as a pharmaceutical composition with a pharmaceutically acceptable excipient and is administered orally, systemically (e.g. transdermally, intranasally or by suppository) or parenterally (e.g. intramuscularly [im], intravenously [iv] or subcutaneously [sc]). The compounds of the invention can thus be administered in a composition that is a semisolid, powder, aerosol, solution, suspension or other appropriate composition, as discussed hereinafter.

A pharmaceutical composition comprises a compound of Formula I, wherein each substituent is defined hereinabove, preferably in combination with a pharmaceutically acceptable excipient. Such excipient is one that is non-toxic and acts to aid in the administration of the compound of this invention. Such excipient may be any solid, liquid, gaseous (in case of an aerosol) or semisolid excipient that is generally available to one of skill in the art and that does not adversely affect the activity of the active agent.

In general, the pharmaceutical composition of this invention will contain a therapeutically effective amount of a compound in combination with at least one excipient. Depending on the type of formulation, size of a unit dosage, kind of excipients and other factors known to those of skill in the art of pharmaceutical

sciences the amount of compound of this invention may vary over a wide range in the composition. In general, the final composition will comprise about 0.001%w to about 99.5%w of a compound of the invention with the remainder being the excipient or excipients. Preferably the level of active compound will be about 0.01%w to about 10.0% and most preferably about 0.1%w to about 1.0%w, with the remainder being a suitable excipient or excipients.

Useful pharmaceutical excipients for the preparation of the pharmaceutical compositions hereof can be solids, semisolids, liquids, or gases. Thus, the compositions can take the form of tablets, pills, capsules, powders, sustained release formulations, solutions, suspensions, elixirs, aerosols, and the like. Solid pharmaceutical excipients include starch, cellulose, talc, glucose, lactose, sucrose, gelatin, malt, rice, flour, chalk, silica gel, magnesium stearate, sodium stearate, glycerol monostearate, sodium chloride, dried skim milk, and the like. Liquid and semisolid excipients may be selected from water, ethanol, glycerol, propylene glycol, various oils, including those of petroleum, animal, vegetable or synthetic origin, for example, peanut oil, soybean oil, mineral oil, sesame oil, and the like. Water, saline, aqueous dextrose, and glycols are preferred liquid carriers, particularly for injectable solutions. Compressed gases are frequently used to dispense the active ingredient in aerosol form. Inert gases suitable for this purpose include nitrogen, carbon dioxide, nitrous oxide, etc. Other suitable pharmaceutical carriers and their formulations are described in "Remington's Pharmaceutical Sciences" by E. W. Martin.

Preferably the pharmaceutical composition is administered in a single unit dosage form for continuous treatment or in a single unit dosage form ad libitum when relief of symptoms is specifically required.

## Presently Preferred Embodiments

While the broadest definition of this invention is set forth in the Summary of the Invention as a compound of Formula I wherein each of X, Y, Z, $R^1$, $R^2$, $R^3$, p, n and t is defined in its broadest aspect, certain compounds of the invention are preferred. For example, the compounds of Formula I wherein X and Y are independently selected from hydrogen, halogen, nitro, amino-carbonyl, amino or lower alkylamino, p is zero and, in case $R^1$ comprises a bicyclic substituent with $R^2$ such $R^2$ is lower alkyl. Of this subgroup those of particular interest include compounds of Formula I wherein $R^2$ is methyl and Z is an oxo group, with particularly preferred compounds being those wherein $R^1$ is 1-azabicyclo[2.2.2]oct-3-yl, 1-azabicyclo-[2.2.2]oct-4-yl, *endo*-9-methyl-9-azabicyclo[3.3.1]non-3-yl, *exo*-9-methyl-9-azabicyclo[3.3.1]non-3-yl, *endo*-8-methyl-8-azabicyclo[3.2.1]oct-3-yl, *exo*-8-methyl-8-azabicyclo[3.2.1]oct-3-yl, *endo*-1-azabicyclo[3.3.1]non-4-yl or *exo*-1-azabicyclo[3.3.1]non-4-yl. Representative compounds are made by following the procedures set out in Examples 1, 2, 3, 5, 6, 17, 19, 20, 21, and 22.

Another subgroup of particular interest includes compounds of Formula I wherein Z is two hydrogens, X and Y are halogen, nitro or amino, p is zero and, in case $R^1$ comprises a bicyclic substituent with $R^2$ such $R_2$ group $R^2$ is methyl. Particularly preferred compounds being those wherein $R^1$ is 1-azabicyclo[2.2.2]oct-3-yl, 1-azabicyclo[2.2.2]oct-4-yl, *endo*-9-methyl-9-azabicyclo[3.3.1]non-3-yl, *exo*-9-methyl-9-azabicyclo[3.3.1]-non-3-yl, *endo*-8-methyl-8-azabicyclo[3.2.1]oct-3-yl, *exo*-8-methyl-8-azabicyclo[3.2.1]oct-3-yl, *endo*-1-azabicyclo[3.3.1]non-4-yl or *exo*-1-azabicyclo[3.3.1]non-4-yl. Representative compounds are made by following the procedure set forth in Examples 4 and 18.

More preferred compounds are the compounds of Formula I wherein X and Y are independently selected from hydrogen, chloro or amino, Z is an oxo group and $R^1$ is 1-azabicyclo[2.2.2]oct-3-yl or *endo*-8-methyl-8-azabicyclo[3.2.1]oct-3-yl and their salts, in particular: S-2-(1-azabicyclo[2.2.2]oct-3-yl)-2,3-dihydro-1H-benz[de]isoquinoline-3-one, S-6-amino-5-chloro-2-(1-azabicyclo[2.2.2]oct-3-yl)-2,3-dihydro-1H-benz[de]-isoquinoline-1-3-dione, S-6-amino-2-(1-azabicyclo[2.2.2]oct-3-yl)-2,3-dihydro-1-H-benz[de]isoquinoline-1-3-dione , S-6-chloro-2-(1-azabicyclo[2.2.2]oct-3-yl-2,3-dihydro-1-H-benz[de]isoquinoline-1-3-dione, and S-6-amino-5-chloro-2-(*endo*-8-methyl-8-azabicyclo[3.2.1]oct-3-yl)-2,3-dihydro-1H-benz[de]isoquinoline-1-3-dione.

It is understood that these subgroups of special interest are particularly useful in the pharmaceutical compositions and methods of treatment of this invention.

## Processes for Preparing Compounds of the Invention

The compounds of Formula I are prepared by a variety of methods applicable to the preparation of these compounds. The synthetic approaches are apparent from the numbered dotted lines (1 to 5') in Formula I below. The dotted lines point schematically to the respective reaction sites and the ensuing table gives a brief description of the various methods that will be described in more detail below. The last column in the table and the letter symbols in parentheses refer to the respective step in the process claim(s).

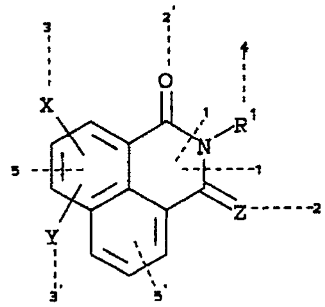

I

| Approach | Method | Step |
|---|---|---|
| 1 | Condensation | a) |
| 2,2' | Reduction or Oxidation | b) |
| 3,3' | Introduction, Removal, Elaboration or Conversion of Substituents, Salt Formation or Conversions | c),d) h),i) |
| 4 | N-Oxidation or Amine Oxide Reduction, Salt Formation or Conversions | e),f) h),i) |
| 5,5' | Aromatization | g) |

Accordingly, the process for preparing the compounds of Formula I comprises one or more of the following steps:

a) the condensation of a 1,8-naphthalic anhydride of the Formula II below with an amine of the formula $R^1NH_2$ wherein $R^1$ has the above meanings or the intramolecular condensation of a compound of the Formula III (IIIA or IIIB) below;

b) the reduction of an isoquinoline-1,3-dione of the Formual IA below to an isoquinoline-3-one of the Formula IB below or the oxidation of a compound of the Formula IB to a compound of the Formula IA below;

c) the introduction or removal of a substituent X or Y in a compound of the Formula I to produce another compound of the Formula I;

d) the conversion of one substituent X or Y into another substituent X or Y;

e) the oxidation of a compound of the Formula I wherein p is zero to the corresponding N-oxide of the compound of the Formula I;

f) the reduction of an N-oxide of the compound of the Formula I to the corresponding compound of the Formula I wherein p is zero;

g) the aromatization of the ring defined by carbon atoms 6a, 7, 8, 9, 9a and 10 to afford a compound of the Formula I;

h) the conversion of a salt of a compound of the Formula I to the corresponding free compound or of a free compound of the Formula I to the corresponding salt;

i) the conversion of a salt of a compound of the Formula I to another salt of the compound of the Formula I;

j) the separation of a mixture of isomers or diastereomers derived from a compound of Formula I into a single isomer or diastereomer; or

k) the stereoselective performance of any of the steps a) through j) with optically active reactants.

A typical condensation step, by which the compounds of Formula I are prepared is described in more detail by the reaction sequence shown below in Reaction Scheme I.

REACTION SCHEME I

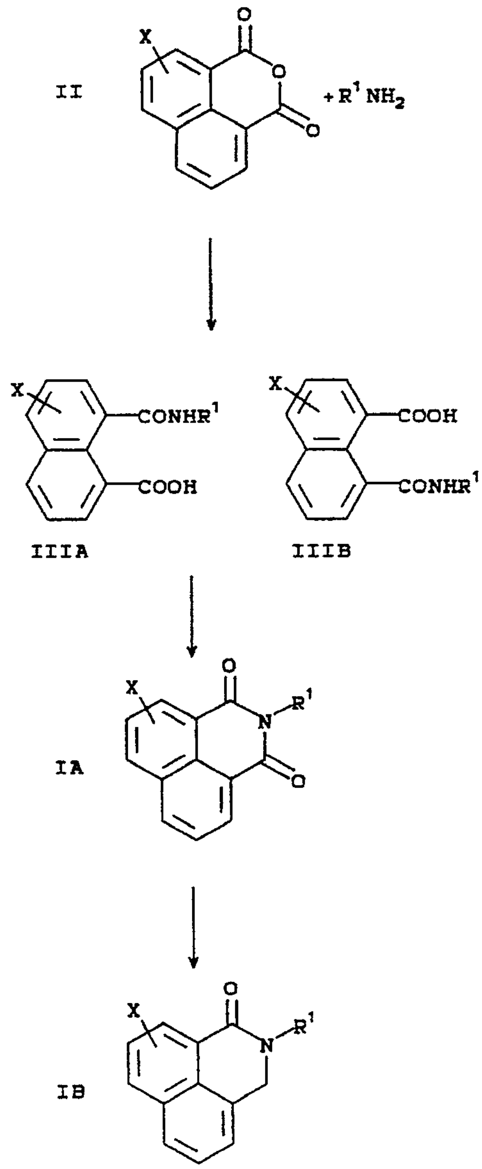

II

IIIA IIIB

IA

IB

wherein X and $R_1$ are as broadly defined above in the Summary of the Invention. The reaction can be also carried out with compounds of the Formulae IIIA or IIIB or reactive derivatives thereof which contain a second substituent Y in the naphthalene ring, but the process is preferably carried out with monosubstituted or unsubstituted 1,8-naphthalic anhydrides. Most preferred is the reaction with unsubstituted anhydrides, or with substituted anhydrides with halogen or nitro substituents which can be converted to other substituents such as amino. Mixed anhydrides derived from the compounds of Formulae IIIA or IIIB and an alkanoic acid may also be employed. Other reactive derivatives may be acid halides, such as acid chlorides, or lower alkanol esters, such as ethyl esters.

Specifically, the compounds of Formula I are prepared by heating a mixture of an optionally substituted 1,8-naphthalic anhydride and an appropriate amine in a suitable solvent at 80 to 200°C and ambient pressure until completion or no further reaction occurred, as for example determined by t.l.c. analysis. Representative, suitable solvents include hydrocarbons, aromatic hydrocarbons such as xylene, toluene, tetralin or biphenyl, alcohols such as methanol, ethanol, n-butanol, t-butanol or isoamylalcohol and ethers such as 1,4-dioxane, pentylether, diglyme (2-methoxyethyl ether) or diphenylether.

Formation of a product with the structure of Formula IA is enhanced by dehydrating agents or activating

agents. This is accomplished by direct addition of the dehydrating agent to the reaction mixture or, alternatively, by isolating the intermediates of Formulae IIIA and IIIB and then treating those intermediates with a dehydrating agent or activating agent in a separate reaction. Representative, suitable dehydrating agents include acetic anhydride, trifluoroacetic anhydride, thionyl chloride, dicyclohexylcarbodiimide (DCC), triethylorthoformate and carbonyldiimidazole and a mixture of DCC and 1-hydroxybenzotriazole.

In general, the starting materials utilized in Scheme I are themselves commercially available or the preparation thereof is known to those of ordinary skill in the art. For example optionally substituted 1,8-naphthalic anhydrides may be prepared by oxidation of the correspondingly substituted or unsubstituted acenaphthene (Dashevskii, M.M. and Karishn, V.P., Org. Chim. Ind., 4, 406 (1937); Graebe, C., Annalen, 327, 77, (1903)).

Amines of the formula $R^1NH_2$ that are useful in this step include those wherein $R^1$ is as defined in the Summary of the Invention section of this application. Particularly useful are the amines wherein $R^1$ is one of the following radicals:
1-azabicyclo[2.2.2]oct-3-yl;
1-azabicyclo[2.2.2]oct-4-yl;
*endo*-9-methyl-9-azabicyclo[3.3.1]non-3-yl;
*exo*-9-methyl-9-azabicyclo[3.3.1]non-3-yl;
*endo*-8-methyl-8-azabicyclo[3.2.1]oct-3-yl;
*exo*-8-methyl-8-azabicyclo[3.2.1]oct-3-yl;
*endo*-1-azabicyclo[3.3.1]non-4-yl; or
*exo*-1-azabicyclo[3.3.1]non-4-yl

Compounds of Formula I wherein Z is two hydrogens (a compound of Formula IB) are prepared by reducing the carbonyl group at the 3-position or 1-position on a corresponding benz[de]isoquinoline-1,3-dione.

The reduction of the carbonyl group may be carried out by conventional methods. Preferred reducing agents are Zn-Hg, Zn/HCl, $B_2H_6$; most preferred are $LiAlH_4(AlCl_3)$, $LiBH_4$, $Zn(BH_4)_2$, $NaBH_4$, in particular $KBH_4$. The reduction can be also carried out by electrolysis. For example, the reduction with $NaBH_4$ or $Zn(BH_4)_2$ is described in Bull. Soc. Chem. Jpn., 61, 2238-2240 (1988).

Compounds of Formula I wherein Z is an oxo group can be prepared by oxidation. This involves oxidizing the corresponding benz[de]isoquinolinone. The oxidation can be carried out by conventional methods with an oxygen source at room temperature or at elevated temperatures. Preferred oxygen sources are oxygen containing gases or pure oxygen. Typically, the oxidation will be carried out with a solution of the benz[de]isoquinolinone, and the oxidizing gas will be bubbled through the solution. Preferred solvents are water, lower alkanols, mixtures of alkanols or aqueous alkanols, such as aqueous methanol, ethanol, n-propanol, n-butanol, etc.

Compounds of Formula I may also be prepared by the reaction sequences shown below in Reaction Scheme II which describes typical examples for the introduction or removal or conversion of substituents.

It is understood that the introduction, removal or conversion of substituents X and Y can be effected by various methods well known to the man skilled in the art. Such well-known methods include electrophilic substitutions with the attacking species being a positive ion or the positive end of a dipole or induced dipole. The leaving group (electrofuge) must necessarily depart without its electron pair. The most common leaving group is the proton. Typical electrophilic substitutions would be halogenation with bromine, or chlorine in the presence of ferric salts. Another electrophilic substitution would be the introduction of the lower alkyl substitent by a Friedel Crafts reaction or nitration. For the preparation of the compounds wherein X is aminocarbonyl the Gattermann amide synthesis with carbamoyl chloride can be employed.

While nucleophilic substitutions are less useful than electrophilic substitutions hyroxydeamination via diazonium salts can be used for the preparation of compounds of the Formula I containing a hydroxy substituent, alkoxydehalogenation for the preparation of compounds with X or Y being alkoxy and aminodehalogenation for the preparation of compunds with X or Y being amino. Nitro or halo compounds can be converted into lower alkylamino, lower dialkylamino or lower alkoxy compounds. Reaction Scheme II describes but a few illustrations of the many methods applicable to the preparation of the compounds of Formula I. Detailed literature references for the preparation of many compounds of the Formula I can be found in Jerry March, Advanced Organic Chemistry, 3rd edition, 1985.

## REACTION SCHEME II

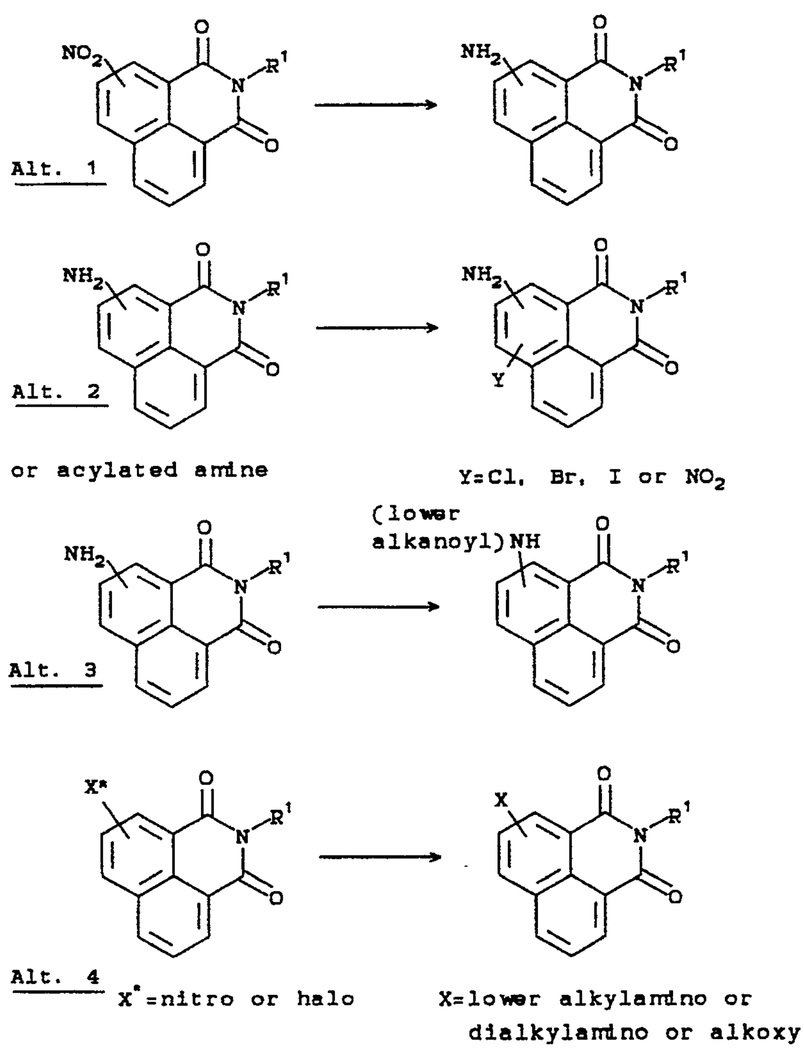


The substituents present on compounds of the Formula I can be reacted with or exchanged to produce additional compounds of the invention. Compounds wherein substituents X and/or Y are $NH_2$ may be produced by the reduction of the corresponding nitro substituents (Alt. 1 of Scheme II) or wherein Y is Cl, Br, I or $NO_2$ by the introduction of such substituent onto a ring that is activated by a X substituent such as $NH_2$, (lower alkyl)amino, di(lower alkyl)amino, OH or alkoxy (Alt. 2 of Scheme II) or wherein X and/or Y is an acylamido substituent by the acylation of the corresponding amino substituent (Alt. 3 of Scheme II). Furthermore, compounds wherein X and/or Y is alkoxy or lower alkyl- or dialkylamino may be produced by substitution of the corresponding nitro or halo substituent (Alt. 4 of Scheme II).

The conversion of the halo- or nitro-substituted benzisoquinolines of Formula I to alkoxy-substituted benzisoquinolines can be effected with the corresponding alkali alcoholate following one of the methods described in J. Org. Chem. (1958), 23, 1700; J.A.C.S. (1964), 86, 3072; Org. Synthesis (1965), 45, 89; J. Org. Chem. (1968), 33, 259; J. Chem. Soc. C (1969), 312. These methods involve the use of alkali metals or alkali amides for the preparation of the alkoholates, i.e. using anhydrous alkanols such as methanol, ethanol or tertiary butanol. The preparation of the alkoxy compounds may be carried out in a stoichiometric excess of the alkanol or ethers such as diethyl ether, dimethylsulfoxide or amines such as collidine. Alternatively,

the halo-substituted benzisoquinolines of Formula I can be converted to Grignard reagents which can be reacted with tertiary butyl esters such as benzoic acid tertiary butyl ester to obtain tertiary butyloxy benzisoquinolines in accordance with the methods described in J.A.C.S. (1959), 81, 4230 and Org. Synthesis (1963), 43, 55. It is preferred to carry out the Grignard formation in ethereal solvents such as diethyl ether or tetrahydrofuran.

The conversion of nitro-substituted compounds of Formula IA to lower alkylamino or di(lower)alkylamino substituted compounds of Formula I can be effected by nucleophilic displacement of the nitro group following the procedure described in Tetrahedron Letters, Vol. 22, No. 24, pp 2303-2306 (1981). The amine is generally applied in a stoichiometric excess at room temperature to elevated temperatures in the presence of an aprotic solvent. Acidic and thermal treatment yields the alkylamino or dialkylamino-substituted compound.

The acylation of compounds of the Formula I with X or Y being an amino group is carried out by conventional acylation with acyl halides, anhydrides, esters or with acids. The conditions follow the well-known Schotten-Baumann procedure with the addition of aqueous alkali to capture liberated hydrogen halid if an acid halide is employed. If acids are being used, the reaction is made to proceed with dehydrating agents such as DCC, or N,N'-carbonyl-diimidazole.

Preparation of Isomers

From the Formula (I) it is apparent that some of the compounds of the invention may have one asymmetric carbon atom (chiral center).

Some $R^1$ substituents, for example, the 1-azabicyclo [2.2.2]oct-4-yl, endo-8-methyl-8-azabicyclo[3.2.1] oct-3-yl, exo-8-methyl-8-azabicyclo[3.2.1]oct-3-yl and the endo-9-methyl-9-azabicyclo[3.3.1]non-3-yl substituents have no asymmetric carbon atom (center of chirality). Therefore, the compounds of Formula I containing an achiral $R^1$ substituent are achiral compounds.

For the compounds of Formula I which have one asymmetric carbon atom, two enantiomeric forms exist, the (R)- and (S)- form as determined by the rules of Cahn et al.

A number of methods suitable for the resolution of enantiomers can be used but the preferred methods depend on the preparation of diastereomeric compounds derived from the enantiomers, in particular enantiomers of $R^1NH_2$ which are used in the condensation step as optically active reagents. While the resolution can be achieved with covalent diastereomeric compounds derived from the compounds of Formula I and diastereomeric complexes, dissociable diastereomeric compounds can also be used. In general, the covalent diastereomers are separated by chromatography but preferred are separation/resolution techniques depending on differences in solubility.

In one method the compounds of Formula I with one asymmetric carbon atom are separated by the formation of crystalline diastereomeric salts between the racemic substrate (R, S) and an optically active acid. Examples of suitable resolving agents which form dissociable salts with the enantiomers of formula I are tartaric acid, o-nitrotartranilic acid, mandelic acid, malic acid, 2-phenoxypropionic acid, hydratropic acid and 2-arylpropionic acids in general, or camphorsulfonic acid. Alternatively, selective crystallization, direct crystallization or chromotography can be used. Specifics of the resolution techniques applicable to the preparation of enantiomers of the Formula I are described in Jean Jacques, André Collet, Samuel H. Wilen, Enantiomers, Racemates and Resolutions, John Wiley & Sons, Inc. (1981).

Alternatively, the compounds of the invention may be prepared using optically active reactants. For example, using (R)-or (S)-amines of the Formula $R^1NH_2$ (wherein $R^1$ has the above meanings) individual isomers of the Formula I may be prepared. This is shown by Examples 2 to 5, 17, 18, 19 and 21 and is currently the most preferred method.

The stereoconfiguration at the chiral centers of the compounds of Formula I can be assigned by circular dichroism, preferably by Single Crystal X-Ray Analysis.

Aromatization

The compounds of Formula I can also be prepared by aromatization of the ring defined by carbon atoms 6a, 7, 8, 9, 9a and 10. The aromatization is relatively easily accomplished because this ring is fused to another aromatic ring, namely the ring defined by carbon atoms 3a, 4, 5, 6, 6a and 10. Many substituent groups X and Y can be present without interference. The aromatization is generally effected by three types of reagents:

1. Hydrogenation catalysts, that is actually dehydrogenation catalysts, such as platinum, palladium, nickel, rhodium, etc. The substrate is heated with the catalyst to relatively high temperatures at about

250 to 370°C. The reaction can often be carried out under milder conditions if a hydrogen acceptor, such as olefines or other aromatic substances are present. As olefines serve maleic acid, cyclohexene, cycloheptenes or cyclopentenes. As aromatic compounds serve benzene, substituted benzenes such as toluene, xylenes; or naphthalene. The acceptor is reduced to the corresponding saturated compound.

2. Reducing elements, such as sulphur or selenium which combine with the hydrogen evolved to give, respectively, hydrogen sulfide or hydrogen selenide.

3. Reducing agents of the quinone type which become reduced to the corresponding hydroquinones. The most important quinones used for aromatization are chloranil (2,3,5,6-tetrachloro-1,4-benzoquinone) and DDQ (2,3-dichloro-5,6-dicyano-1,4-benzoquinone). The latter is more reactive and may be used in cases where the substrate is difficult to dehydrogenate. The mechanism probably involves the transfer of hydride to the quinone oxygen, followed by the transfer of a proton to the phenolate anion.

Other reagents that may be used are atmospheric oxygen, manganese dioxide, selenium dioxide and various strong bases, triphenylcarbinol in trifluorophenylacetic acid, and activated charcoal. In some instances the hydrogen is not released as gas or transferred to an external oxidizing agent but instead serves to reduce another substrate. Detailed literature references can be found in Jerry March, Advanced Organic Chemistry, 3rd Edition, 1985.

The starting materials that may be aromatized have the following Formula (IV)

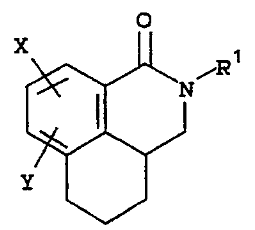

(IV)

wherein X, Y and $R^1$ have the above meanings. These compounds are prepared by the methods disclosed in Case 26890 filed November 1990. Specifically, the preparation of the compounds of Formula (IV) involves the formylation of a compound of the Formula (V)

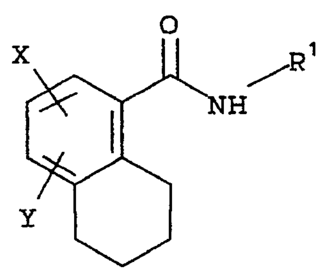

(V)

wherein X, Y and $R^1$ have the above meanings with a formaldehyde source such as dialkylformamides, in particular, dimethylformamide. In this step intermediate amide of the Formula V is reacted with a formylating agent in the presence of a strong base. The reaction is carried out in a non-reactive ethereal solvent such as diethyl ether, dimethoxyethane or tetrahydrofuran (THF), the last being preferred. The formylating agent useful for this reaction is any compound that achieves reaction of the amide of Formula II with the formyl group (-CH=O), particularly a dialkylformamide, such as dimethyl formamide (DMF), diethyl formamide, etc., a N-aryl-N-alkylformamide, such as N-phenyl-N-methylformamide, etc. The formylating agent is generally used in molar excess relative to the amide V, for example at a ratio of about 1.1 to about 5.0, with 1.5 to 2.5 being preferred. The strong base useful in this reaction is one that aids the progression of the reaction and can be any appropriate alkyllithium or Grignard reagent. *n*-Butyllithium is particularly useful because of its availability. In general, the reaction takes place under an inert atmosphere (e.g. argon) to prevent the oxidation of the alkyllithium and at a temperature range of about -70°C to ambient temperature. Preferably the temperature is about -20°C to about 0°C, as the reduced temperature is thought to stabilize the intermediate anions formed in this step.

One of ordinary skill in the art will also recognize that a compound of Formula I may be prepared as an acid addition salt or as the corresponding free base. If prepared as an acid addition salt, the compound is converted to the free base by treatment with a suitable base such as ammonium hydroxide solution, sodium hydroxide or the like. When converting a free base to an acid addition salt, the compound is reacted with a suitable organic or inorganic acid.

It is also understood that compounds of this invention that are the *N*-oxides of compounds of Formula I (the *N*-oxides of the cyclic amine portion of $R^1$) are prepared by means known in the art by reacting a compound of Formula I with an oxidizing agent such as pertrifluoroacetic acid, permaleic acid, perbenzoic acid, peracetic acid, *m*-chloroperoxybenzoic acid. With m-chloroperoxybenzoic acid the oxidation is conducted under cooling in an inert, organic solvent such as a halogenated hydrocarbon, e.g. dichloromethane. For this oxidation to take place effectively, the compound of Formula I is preferably in the free base form.

The N-oxides of the compounds of Formula I can also be reduced by methods known in the art. A number of reducing agents are suitable for this purpose, specifically sulfur dioxide, sulfur itself, triaryl phosphines such as triphenyl phosphine, alkali boranates such as lithium or sodium boranate, or phosphorous trichloride or tribromide. The reaction will be conducted at a temperature between 0 and 80 °C, with gradual raising of the temperature and the reaction mixture is occasionally shaken. As some of the N-oxides have a low melting point the reduction can be conducted without an additional solvent. If a solvent is being used then the following solvents are preferred: acetonitrite, ethanol, or aqueous dioxane.

Because of the hazardous nature of many of the reducing agents or of the reaction products, the preparation should be conducted in a closed system to avoid exposure to irritating fumes.

The compounds of Formula I with X or Y being hydroxy, nitro, or amino substituents can be converted to other X or Y substituents in a manner known per se. The nitro group can be converted to an amino group by a number of well-described methods, either metallic reducing agents such as zinc, tin or iron and acid, catalytic hydrogenation, sulfides such as sodium hydrogensulfide, ammonium sulfide, complexes of aluminum trihydride with aluminum chloride or hydrazine and a catalyst. Specifically useful methods are described by Joffe, Tartakovskii, and Novikov, Russ. Chem. Rev. 35, 19-32 (1966).

The resulting amino compounds in turn can be alkylated or acylated. The alkylation is conducted with alkyl halides, sulfates, such as dimethyl sulfate or sulfonates. The amino compounds can also be acylated in a manner known per se with acyl halides, anhydrides, esters or with acids. The conditions follow that of the well-known Schotten-Baumann procedure. Frequently aqeuous alkali is added to combine with the liberated hydrogen halide. If acids are being used the reaction is made to proceed in good yield at room temperature with dehydrating agents such as dicyclohexylcarbodiimide or N,N'-carbonyl diimidazole. The acylation can also be carried out as anhydrous coupling of the acid chloride with the free base in a suitable organic solvent (toluene, tetrahydrofuran, ethyl acetate) with good mechanical stirring.

The compounds of Formula I with $R^1$ being alkoxy can also be prepared from the compounds with $R^1$ being a hydroxy group by alkylation. The reaction proceeds quickly with diazo compounds such as diazomethane under mild conditions with high yields. The best method, however, is the Williamson reaction which is carried out in the presence of a base.

The compounds of Formula I with X or Y being hydroxy are prepared by dealkylation or debenzylation of starting materials of the Formula I with X or Y being either alkyloxy or benzyloxy.

The dealkylation is carried out preferably by acid cleavage of the alkyl aryl ether, preferably with hydrogen iodide or hydrogen bromide. Other suitable cleaving agents are Lewis acids such $BF_3$, $BCl_3$, $BBr_3$ or $AlCl_3$ or anhydrous sulfonic acids or Grignard reagents. If HBr or HJ are used the reaction is generally carried out with the acid addition salt of the alkyl aryl ether using an excess of cleaving agent without a solvent at elevated temperatures, i.e., 40 °C to the boiling point of the reaction mixture, preferably 60 to 95 °C.

The debenzylation is carried out around room temperature under hydrogen with a palladium, platinum or rhodium catalyst with the benzyl aryl ether in solution in an inert solvent. Frequently the catalyst is removed by filtration for recovery and regeneration.

In summary the therapeutically active compounds of this invention are preferably prepared by

(1) reacting a mixture of an optionally substituted 1,8-naphthalic anhydride and an appropriate amine in with a dehydrating agent until a product with the structure of Formula I is formed,

(2) reacting or exchanging substituents present in compounds of Formula I to yield additional substituted products,

(3) reducing one of the carbonyl groups present in compounds of Formula I to yield the corresponding benz[de]isoquinoline-3-one,

(4) converting an acid addition salt of a compound of Formula I to the corresponding free base,

(5) converting a free base of a compound of Formula I to form the corresponding pharmaceutically

acceptable salt,

(6) oxidizing a compound of Formula I to form the corresponding *N*-oxide of the $R^1$ component of Formula I, or

(7) separating a mixture of isomers of a compound of Formula I into a single isomer.

In any of the above last step processes, a reference to Formula I refers to such Formula wherein X, Y, Z, $R^1$, $R^2$, $R^3$, p, n and t are as defined in their broadest definitions set forth in the Summary of the Invention, with the processes applying particularly to the presently preferred embodiments.

EXAMPLE 1

The following is an example of Scheme I in the synthesis of *RS*-2-(1-azabicyclo[2.2.2]oct-3-yl)-2,3-dihydro-1*H*-benz[*de*]isoquinoline-1,3-dione, a compound with the structure of Formula I wherein Z is oxygen, X and Y are hydrogen and $R^1$ is chosen from subformula (b) wherein n is 2 and p is 0.

A. 1.9 Grams (9.6 mmoles) of 1,8-naphthalic anhydride was reacted with 1.2 grams (9.5 mmoles) of *RS*-3-aminoquinuclidine in 100 ml of n-butanol for 3 hours at reflux temperature. The solution was concentrated to dryness and the remaining solid was purified by passage through silica-gel (5-10% gradient of methanol in $CH_2Cl_2$ and approximately 1% aqueous $NH_4OH$). 2.45 Grams of *RS*-2-(1-azabicyclo[2.2.2]oct-3-yl)-2,3-dihydro-1*H*-benz[*de*]isoquinoline-1,3-dione, m.p. 206-207° C remained after evaporation of the solvent. This compound was dissolved in 40 ml of hot ethanol and a few ml of ethanol containing approximately 0.4 Grams of HCl was added to give *RS*-2-(1-azabicyclo[2.2.2]oct-3-yl)-2,3-dihydro-1*H*-benz[*de*]isoquinoline-1,3-dione HCl, m.p. 340-342° C.

B. Proceeding as in Part A, but replacing *RS*-3-aminoquinuclidine with *S*-3-aminoquinuclidine, *S*-2-(1-azabicyclo[2.2.2]oct-3-yl)-2,3-dihydro-1*H*-benz[*de*]isoquinoline-1,3-dione, m.p. 185-189° C $[\alpha]_D^{25}$ -73.8° (c 0.4, $CHCl_3$) and *S*-2-(1-azabicyclo[2.2.2]oct-3-yl)-2,3-dihydro-1*H*-benz[*de*]isoquinoline-1,3-dione hydrochloride, m.p. 323-324° C, $[\alpha]_D^{25}$ -30.6° (c 0.565, MeOH) were prepared.

Proceeding as in Part A, but replacing 1,8-naphthalic anhydride with 4-bromo-1,8-naphthalic anhydride, *RS*-6-bromo-2-(1-azabicyclo[2.2.2]oct-3-yl)-2,3-dihydro-1*H*-benz[*de*]isoquinoline-1,3-dione, m.p. 305-308° C was prepared.

Proceeding as in Part A, but replacing, 1,8-naphthalic anhydride with 4-chloro-1,8-naphthalic anhydride, *RS*-6-chloro-2-(1-azabicyclo[2.2.2]oct-3-yl)-2,3-dihydro-1*H*-benz[*de*]isoquinoline-1,3-dione, m.p. 226-229° C and *RS*-6-chloro-2-(1-azabicyclo[2.2.2]oct-3-yl)-2,3-dihydro-1*H*-benz[*de*]isoquinoline-1,3-dione hydrochloride, m.p. 350-352° C were prepared.

Proceeding as in Part A compounds that may be prepared include:

2-(1-azabicyclo[2.2.2]oct-4-yl)-2,3-dihydro-1*H*-benz[*de*]isoquinoline-1,3-dione;

2-(*endo*-9-methyl-9-azabicyclo[3.3.1]non-3-yl)-2,3-dihydro-1*H*-benz[*de*]isoquinoline-1,3-dione;

2-(*exo*-9-methyl-9-azabicyclo[3.3.1]non-3-yl)-2,3-dihydro-1*H*-benz[*de*]isoquinoline-1,3-dione;

2-(*endo*-8-methyl-8-azabicyclo[3.2.1]oct-3-yl)-2,3-dihydro-1*H*-benz[*de*]isoquinoline-1,3-dione;

2-(*exo*-8-methyl-8-azabicyclo[3.2.1]oct-3-yl)-2,3-dihydro-1*H*-benz[*de*]isoquinoline-1,3-dione;

2-(*endo*-1-azabicyclo[3.3.1]non-4-yl)-2,3-dihydro-1*H*-benz[*de*]isoquinoline-1,3-dione;

2-(*exo*-1-azabicyclo[3.3.1]non-4-yl)-2,3-dihydro-1*H*-benz[*de*]isoquinoline-1,3-dione.

EXAMPLE 2

The following is an example of Scheme I in the synthesis of *S*-6-nitro-2-(1-azabicyclo[2.2.2.]oct-3-yl)-2,3-dihydro-1*H*-benz[*de*]isoquinoline-1,3-dione, a compound with the structure of Formula I wherein the Z is oxygen, X is nitro in the 6-position, Y is hydrogen and $R^1$ is chosen from subformula (b) wherein n is 2 and p is 0.

A. A solution of 3.1 grams (24.6 mmoles) of composition *S*-3-aminoquinuclidine in 40 ml of xylenes (~ 40% of meta and 20% of each of the ortho- and para-isomers and ethylbenzene) was added dropwise to a boiling solution of 6.1 grams (25.1 mmoles) of 4-nitro-1,8-naphthalic anhydride. The reaction mixture was kept at reflux temperature for 6 hours while the water that formed was collected in a Dean-Stark trap. Some acetic anhydride (1.5 ml or 13.0 mmoles) was added and heating was continued for 16 hours. The solvent was removed by evaporation under vacuum and the remaining crude product was purified by column chromatography (silica-gel of 5% gradient of methanol in $CH_2Cl_2$ and approximately 1% aqueous $NH_4OH$). 6.0 grams of *S*-6-nitro-2-(1-azabicyclo[2.2.2.]oct-3-yl)-2,3-dihydro-1*H*-benz[*de*]-isoquinoline-1,3-dione, m.p. 214-219° C remained.

Proceeding as in Part A, but replacing *S*-3-aminoquinuclidine with *RS*-3-aminoquinuclidine, *RS*-6-nitro-2-(1-azabicyclo[2.2.2.]oct-3-yl)-2,3-dihydro-1*H*-benz[*de*]isoquinoline-1,3-dione, m.p. 224-226° C and *RS*-6-nitro-2-(1-azabicyclo[2.2.2.]oct-3-yl)-2,3-dihydro-1*H*-benz[*de*]isoquinoline-1,3-dione hydrochloride, m.p. >330° C) were prepared.

Proceeding as in Part A, but replacing *S*-3-aminoquinuclidine with *R*-3-aminoquinuclidine, *R*-6-nitro-2-(1-azabicyclo[2.2.2.]oct-3-yl)-2,3-dihydro-1*H*-benz[*de*]isoquinoline-1,3-dione; m.p. 216-220° C was prepared.

Proceeding as in Part A compounds that may be prepared include:
6-nitro-2-(1-azabicyclo[2.2.2]oct-4-yl)-2,3-dihydro-1*H*-benz(*de*]isoquinoline-1,3-dione;
6-nitro-2-(*endo*-9-methyl-9-azabicyclo[3.3.1]non-3-yl)-2,3-dihydro-1*H*-benz[*de*]isoquinoline-1,3-dione;
6-nitro-2-(*exo*-9-methyl-9-azabicyclo[3.3.1]non-3-yl)-2,3-dihydro-1*H*-benz[*de*]isoquinoline-1,3-dione;
6-nitro-2-(*endo*-8-methyl-8-azabicyclo[3.2.1]oct-3-yl)-2,3-dihydro-1*H*-benz[*de*]isoquinoline-1,3-dione;
6-nitro-2-(*exo*-8-methyl-8-azabicyclo[3.2.1]oct-3-yl)-2,3-dihydro-1*H*-benz[*de*]isoquinoline-1,3-dione;
6-nitro-2-(*endo*-1-azabicyclo[3.3.1]non-4-yl)-2,3-dihydro-1*H*-benz[*de*]isoquinoline-1,3-dione;
6-nitro-2-(*exo*-1-azabicyclo[3.3.1]non-4-yl)-2,3-dihydro-1*H*-benz[*de*]isoquinoline-1,3-dione.

EXAMPLE 3

The following is an example of Scheme I in the synthesis of *S*-6-nitro-2-(1-azabicyclo[2.2.2.]oct-3-yl)-2,3-dihydro-1*H*-benz[*de*]isoquinoline-1,3-dione, a compound with the structure of Formula I wherein the Z is oxygen, X is nitro in the 6-position, Y is hydrogen and $R^1$ is chosen from subformula (b) wherein n is 2 and p is 0.

A sample of 14.5 Grams (59.6 mmoles) of 4-nitro-1,8-naphthalic anhydride was suspended in 250 ml of toluene at reflux temperature. A solution of 7.5 grams (59.5 mmoles) of *S*-3-aminoquinuclidine in 100 ml of toluene was added in a thin stream. Reflux temperature was maintained for an additional 15 minutes and the reaction was then allowed to cool to ambient temperature. The intermediate(s) *S*-8-(1-azabicyclo[2.2.2.]oct-3-yl)aminocarbonyl)-4 and/or 5-nitro-naphthalene-1-carboxylic acid were collected to yield 18.8 grams of product (m.p. 172-175° C). The intermediate(s) (52.3 mmoles) and 8.7 grams (53.7 mmoles) of carbonyldiimidazole were stirred in 500 ml of $CH_2Cl_2$ for 16 hours. The reaction mixture was washed twice with 200 ml of water containing 5% $NaHCO_3$. The $CH_2Cl_2$ solution was dried over $K_2CO_3$, filtered and concentrated. 5.3g of *S*-6-nitro-2-(1-azabicyclo[2.2.2.]oct-3-yl)-2,3-dihydro-1*H*-benz[*de*]isoquinoline-1,3-dione, m.p. 214-219° C remained.

EXAMPLE 4

The following is an example of Scheme I in the synthesis of *S*-2-(1-azabicyclo[2.2.2]oct-3-yl)-2,3-dihydro-1*H*-benz[*de*]isoquinoline-3-one, a compound with the structure of Formula I wherein Z is two hydrogens, X is hydrogen, Y is hydrogen and $R^1$ is chosen from subformula (b) wherein n is 2 and p is 0.

A. A sample of 1.1 Grams (3.5 mmoles) of *S*-2-(1-azabicyclo[2.2.2.]oct-3-yl)-2,3-dihydro-1*H*-benz[*de*]-isoquinoline-1,3-dione, from Example 2, was added to a solution of 1.3 Grams (34.0 mmoles) of $NaBH_4$ in 110 ml of ethanol and 10 ml of water. After stirring for 5 hours at ambient temperature the solution was concentrated to a small volume, treated with 10% HCl until strongly acidic and stirred for an additional 2 hours. The solution was made alkaline by addition of NaOH and then extracted with $CH_2Cl_2$. The $CH_2Cl_2$ extract was concentrated and the residue was chromatographed on silica-gel (5% methanol in $CH_2Cl_2$ and approximately 1% aqueous $NH_4OH$) to yield *S*-2-(1-azabicyclo[2.2.2]oct-3-yl)-2,3-dihydro-1*H*-benz-[*de*]isoquinoline-3-one, (HCl salt) m.p. 278-279° C, $[\alpha]_D^{25}$ -16.9° (c 0.48, MeOH).

B. Proceeding as in Part A compounds that may be prepared include:
6-amino-5-chloro-2-(1-azabicyclo[2.2.2]oct-4-yl)-2,3-dihydro-1*H*-benz[*de*]isoquinoline-3-one;
6-amino-5-chloro-2-(*endo*-9-methyl-9-azabicyclo[3.3.1]non-3-yl)-2,3-dihydro-1*H*-benz[*de*]isoquinoline-3-one;
6-amino-5-chloro-2-(*exo*-9-methyl-9-azabicyclo[3.3.1]non-3-yl)-2,3-dihydro-1*H*-benz[*de*]isoquinoline-3-one;
6-amino-5-chloro-2-(*endo*-8-methyl-8-azabicyclo[3.2.1]oct-3-yl)-2,3-dihydro-1*H*-benz[*de*]isoquinoline-3-one;
6-amino-5-chloro-2-(*exo*-8-methyl-8-azabicyclo[3.2.1]oct-3-yl)-2,3-dihydro-1*H*-benz[*de*]isoquinoline-3-one;
6-amino-5-chloro-2-(*endo*-1-azabicyclo[3.3.1]non-4-yl)-2,3-dihydro-1*H*-benz[*de*]isoquinoline-3-one;
6-amino-5-chloro-2-(*exo*-1-azabicyclo[3.3.1]non-4-yl)-2,3-dihydro-1*H*-benz[*de*]isoquinoline-3-one;
6-amino-5-iodo-2-(1-azabicyclo[2.2.2]oct-4-yl)-2,3-dihydro-1*H*-benz[*de*]isoquinoline-3-one;
6-amino-5-iodo-2-(*endo*-9-methyl-9-azabicyclo[3.3.1]non-3-yl)-2,3-dihydro-1*H*-benz[*de*]isoquinoline-3-one;
6-amino-5-iodo-2-(*exo*-9-methyl-9-azabicyclo[3.3.1]non-3-yl)-2,3-dihydro-1*H*-benz[*de*]isoquinoline-3-one;
6-amino-5-iodo-2-(*endo*-8-methyl-8-azabicyclo[3.2.1]oct-3-yl)-2,3-dihydro-1*H*-benz[*de*]isoquinoline-3-one;

6-amino-5-iodo-2-(*exo*-8-methyl-8-azabicyclo[3.2.1]oct-3-yl)-2,3-dihydro-1*H*-benz[*de*]isoquinoline-3-one;
6-amino-5-iodo-2-(*endo*-1-azabicyclo[3.3.1]non-4-yl)-2,3-dihydro-1*H*-benz[*de*]isoquinoline-3-one;
6-amino-5-iodo-2-(*exo*-1-azabicyclo[3.3.1]non-4-yl)-2,3-dihydro-1*H*-benz[*de*]isoquinoline-3-one;
6-amino-5-bromo-2-(1-azabicyclo[2.2.2]oct-4-yl)-2,3-dihydro-1*H*-benz[*de*]isoquinoline-3-one;
6-amino-5-bromo-2-(*endo*-9-methyl-9-azabicyclo[3.3.1]non-3-yl)-2,3-dihydro-1*H*-benz[*de*]isoquinoline-3-one;
6-amino-5-bromo-2-(*exo*-9-methyl-9-azabicyclo[3.3.1]non-3-yl)-2,3-dihydro-1*H*-benz[*de*]isoquinoline-3-one;
6-amino-5-bromo-2-(*endo*-8-methyl-8-azabicyclo[3.2.1]oct-3-yl)-2,3-dihydro-1*H*-benz[*de*]isoquinoline-3-one;
6-amino-5-bromo-2-(*exo*-8-methyl-8-azabicyclo[3.2.1]oct-3-yl)-2,3-dihydro-1*H*-benz[*de*]isoquinoline-3-one;
6-amino-5-bromo-2-(*endo*-1-azabicyclo[3.3.1]non-4-yl)-2,3-dihydro-1*H*-benz[*de*]isoquinoline-3-one;
6-amino-5-bromo-2-(exo-1-azabicyclo[3.3.1]non-4-yl)-2,3-dihydro-1*H*-benz[*de*]isoquinoline-3-one.

EXAMPLE 5

The following is an example of Alt. 1 of Scheme II in the synthesis of *S*-6-amino-2-(1-azabicyclo[2.2.2]-oct-3-yl)-2,3-dihydro-1*H*-benz[*de*]isoquinoline-1,3-dione, a compound with the structure of Formula I wherein Z is oxygen, X is amino in the 6-position, Y is hydrogen, and $R^1$ is chosen from subformula (b) wherein n is 2 and p is 0.

A. A solution of 3.2 grams (8.8 mmoles) of *S*-6-nitro-2-(1-azabicyclo[2.2.2.]oct-3-yl)-2,3-dihydro-1*H* -benz-[*de*]isoquinoline-1,3-dione, from Example 2, in 80 ml of acetic acid was hydrogenated at atmospheric pressure over 1.2 grams of 10% Pd/C catalyst for 3 hours. The catalyst was removed by filtration and the filtrate was concentrated to a small volume. Some water was added and the aqueous solution was added dropwise to dilute $NH_4OH$. The solid that precipitated was collected on a Buechner funnel and dried under vacuum to yield 2.57 grams of *S*-6-amino-2-(1-azabicyclo[2.2.2]oct-3-yl)-2,3-dihydro-1*H*-benz[*de*]iso-quinoline-1,3-dione, m.p. 305-308° C, $[\alpha]_D^{25}$ -44.2° (c 0.373, MeOH).

B. Proceeding as in Part A, but replacing *S*-6-nitro-2-(1-azabicyclo[2.2.2.]oct-3-yl)-2,3-dihydro-1*H*-benz-[*de*]isoquinoline-1,3-dione with *RS*-6-nitro-2-(1-azabicyclo[2.2.2.]oct-3-yl)-2,3-dihydro-1*H*-benz[*de*]-isoquinoline-1,3-dione, *RS*-6-amino-2-(1-azabicyclo[2.2.2]oct-3-yl)-2,3-dihydro-1*H*-benz[*de*]iso-quinoline-1,3-dione dihydrochloride, m.p. 308-312° C was prepared.

Proceeding as in Part A, but replacing *S*-6-nitro-2-(1-azabicyclo[2.2.2.]oct-3-yl)-2,3-dihydro-1*H*-benz[*de*]-isoquinoline-1,3-dione with 6-nitro-2-(*endo*-8-methyl-azabicyclo[3.2.1.]oct-3-yl)-2,3-dihydro-1*H*-benz[*de*]-isoquinoline-1,3-dione, 6-amino-2-(*endo*-8-methyl-azabicyclo[3.2.1.]oct-3-yl)-2,3-dihydro-1*H*-benz[*de*]-isoquinoline-1,3-dione dihydrochloride, m.p. 327-330° C was produced.

Proceeding as in Part A, but replacing *S*-6-nitro-2-(1-azabicyclo[2.2.2.]oct-3-yl)-2,3-dihydro-1*H*-benz[*de*]-isoquinoline-1,3-dione with *RS*-5-nitro-2-(1-azabicyclo[2.2.2.]oct-3-yl)-2,3-dihydro-1*H*-benz[*de*]isoquinoline-1,3-dione, *RS*-5-amino-2-(1-azabicyclo[2.2.2]oct-3-yl)-2,3-dihydro-1*H*-benz[*de*]isoquinoline-1,3-dione dihydrochloride, m.p. 287-293° C was prepared. Anal.: Calcd. for $C_{19}H_{21}Cl_2N_3O_2$: C, 57.88; H, 5.73; N, 10.65%. Found: C, 58.03; H, 5.54; N, 10.58%.

EXAMPLE 6

The following is an example of Alt. 2 of Scheme II in the synthesis of *RS*-6-amino-5-chloro-2-(1-azabicyclo[2.2.2.]oct-3-yl)-2,3-dihydro-1*H*-benz[*de*]isoquinoline-1,3-dione, a compound with the structure of Formula I wherein Z is oxygen, X is amino in the 6-position, Y is chloro in the 5-position and $R^1$ is chosen from subformula (b) wherein n is 2 and p is 0.

A. A solution of 0.5 Grams (1.55 mmoles) of *RS*-6-amino-2-(1-azabicyclo[2.2.2.]oct-3-yl)-2,3-dihydro-1*H*-benz[*de*]isoquinoline-1,3-dione, from Example 5, in a mixture of 10 ml acetic acid and 10 ml acetonitrile was cooled in an ice-water bath. A solution containing 1.7-1.8 mmoles of chlorine in acetic acid was added. The reaction was concentrated to a small volume and the product was precipitated by addition of ammonium hydroxide. Purification by column chromatography (10% methanol in $CH_2Cl_2$ and approximately 1% aqueous $NH_4OH$) to yield 240 milligrams of pure *RS*-6-amino-5-chloro-2-(1-azabicyclo[2.2.2.]-oct-3-yl)-2,3-dihydro-1*H*- benz[*de*]isoquinoline-1,3-dione, m.p. >300° C.

B. Proceeding as in Part A, but replacing *RS*-6-amino-2-(1-azabicyclo[2.2.2.]oct-3-yl)-2,3-dihydro-1*H*-benz[*de*]isoquinoline-1,3-dione with *S*-6-amino-2-(1-azabicyclo[2.2.2.]oct-3-yl)-2,3-dihydro-1*H*-benz[*de*]-isoquinoline-1,3-dione, *S*-6-amino-5-chloro-2-(1-azabicyclo[2.2.2.]oct-3-yl)-2,3-dihydro-1*H*-benz[*de*]-isoquinoline-1,3-dione, m.p. 215-219° C, $[\alpha]_D^{25}$ -77° (c 0.645, EtOH) and *S*-6-amino-5-chloro-2-(1-azabicyclo[2.2.2.]oct-3-yl)-2,3-dihydro-1*H*-benz[*de*]isoquinoline-1,3-dione hydrochloride, m.p. 345-349 °C, $[\alpha]_D^{25}$ -34 ° (c 0.47, MeOH) were prepared.

Proceeding as in Part A, but replacing *RS*-6-amino-2-(1-azabicyclo[2.2.2.]oct-3-yl)-2,3-dihydro-1*H*-benz-[*de*]isoquinoline-1,3-dione with *R*-6-amino-2-(1-azabicyclo[2.2.2.]oct-3-yl)-2,3-dihydro-1*H*-benz[*de*]-isoquinoline-1,3-dione, *R*-6-amino-5-chloro-2-(1-azabicyclo[2.2.2.]oct-3-yl)-2,3-dihydro-1*H*-benz[*de*]-isoquinoline-1,3-dione hydrochloride, m.p. 344-347 °C, $[\alpha]_D^{25}$ -42.5 ° (c 0.425, MeOH) was prepared.

Proceeding as in Part A, but replacing chlorine with iodine, *RS*-6-amino-5-iodo-2-(1-azabicyclo[2.2.2.]-oct-3-yl)-2,3-dihydro-1*H*-benz[*de*]isoquinoline-1,3-dione hydroiodide, m.p. 221-223 °C, $[\alpha]_D^{25}$ -33.9 ° (c 0.6, DMSO) was prepared.

Proceeding as in Part A, but replacing chlorine with iodine and *RS*-6-amino-2-(1-azabicyclo[2.2.2.]oct-3-yl)-2,3-dihydro-1*H*-benz[*de*]isoquinoline-1,3-dione with *R*-6-amino-2-(1-azabicyclo[2.2.2.]oct-3-yl)-2,3-dihydro-1*H*-benz[*de*]isoquinoline-1,3-dione, *R*-6-amino-5-iodo-2-(1-azabicyclo[2.2.2.]oct-3-yl)-2,3-dihydro-1*H*-benz-[*de*]isoquinoline-1,3-dione hydrochloride, m.p. 225 °C, $[\alpha]_D^{25}$ +41 ° (MeOH) was prepared.

Proceeding as in Part A compounds that may be made include:

6-amino-5-chloro-2-(1-azabicyclo[2.2.2]oct-4-yl)-2,3-dihydro-1*H*-benz[*de*]isoquinoline-1,3-dione;
6-amino-5-chloro-2-(*endo*-9-methyl-9-azabicyclo[3.3.1]non-3-yl)-2,3-dihydro-1*H*-benz[*de*]isoquinoline-1,3-dione;
6-amino-5-chloro-2-(*exo*-9-methyl-9-azabicyclo[3.3.1]non-3-yl)-2,3-dihydro-1*H*-benz[*de*]isoquinoline-1,3-dione;
6-amino-5-chloro-2-(*endo*-8-methyl-8-azabicyclo[3.2.1]oct-3-yl)-2,3-dihydro-1*H*-benz[*de*]isoquinoline-1,3-dione;
6-amino-5-chloro-2-(*exo*-8-methyl-8-azabicyclo[3.2.1]oct-3-yl)-2,3-dihydro-1*H*-benz[*de*]isoquinoline-1,3-dione;
6-amino-5-chloro-2-(*endo*-1-azabicyclo[3.3.1]non-4-yl)-2,3-dihydro-1*H*-benz[*de*]isoquinoline-1,3-dione;
6-amino-5-chloro-2-(*exo*-1-azabicyclo[3.3.1]non-4-yl)-2,3-dihydro-1*H*-benz[*de*]isoquinoline-1,3-dione;
6-amino-5-iodo-2-(1-azabicyclo[2.2.2]oct-4-yl)-2,3-dihydro-1*H*-benz[*de*]isoquinoline-1,3-dione;
6-amino-5-iodo-2-(*endo*-9-methyl-9-azabicyclo[3.3.1]non-3-yl)-2,3-dihydro-1*H*-benz[*de*]isoquinoline-1,3-dione;
6-amino-5-iodo-2-(*exo*-9-methyl-9-azabicyclo[3.3.1]non-3-yl)-2,3-dihydro-1*H*-benz(*de*]isoquinoline-1,3-dione;
6-amino-5-iodo-2-(*endo*-8-methyl-8-azabicyclo[3.2.1]oct-3-yl)-2,3-dihydro-1*H*-benz[*de*]isoquinoline-1,3-dione;
6-amino-5-iodo-2-(*exo*-8-methyl-8-azabicyclo[3.2.1]oct-3-yl)-2,3-dihydro-1*H*-benz[*de*]isoquinoline-1,3-dione;
6-amino-5-iodo-2-(*endo*-1-azabicyclo[3.3.1]non-4-yl)-2,3-dihydro-1*H*-benz[*de*]isoquinoline-1,3-dione;
6-amino-5-iodo-2-(*exo*-1-azabicyclo[3.3.1]non-4-yl)-2,3-dihydro-1*H*-benz[*de*]isoquinoline-1,3-dione;
6-amino-5-bromo-2-(1-azabicyclo[2.2.2]oct-4-yl)-2,3-dihydro-1*H*-benz[*de*]isoquinoline-1,3-dione;
6-amino-5-bromo-2-(*endo*-9-methyl-9-azabicyclo[3.3.1]non-3-yl)-2,3-dihydro-1*H*-benz[*de*]isoquinoline-1,3-dione;
6-amino-5-bromo-2-(*exo*-9-methyl-9-azabicyclo[3.3.1]non-3-yl)-2,3-dihydro-1*H*-benz[*de*]isoquinoline-1,3-dione;
6-amino-5-bromo-2-(*endo*-8-methyl-8-azabicyclo[3.2.1]oct-3-yl)-2,3-dihydro-1*H*-benz[*de*]isoquinoline-1,3-dione;
6-amino-5-bromo-2-(*exo*-8-methyl-8-azabicyclo[3.2.1]oct-3-yl)-2,3-dihydro-1*H*-benz[*de*]isoquinoline-1,3-dione;
6-amino-5-bromo-2-(*endo*-1-azabicyclo[3.3.1]non-4-yl)-2,3-dihydro-1*H*-benz[*de*]isoquinoline-1,3-dione;
6-amino-5-bromo-2-(*exo*-1-azabicyclo[3.3.1]non-4-yl)-2,3-dihydro-1*H*-benz[*de*]isoquinoline-1,3-dione.

## EXAMPLE 7

### 5-$HT_3$ RECEPTOR SCREENING ASSAY

The following describes an *in vitro* assay for determining the 5-$HT_3$ receptor binding affinity of compounds of Formula I. The method is essentially that described by Kilpatrick et al., previously cited, which measures the affinity for 5-$HT_3$ receptors of the rat cerebral cortex radiolabelled with [$^3$H]quipazine.

Membranes are prepared from the cerebral cortices of rat brains homogenized in 50 mM Tris buffer (pH 7.4 at 4 °C) using a Polytron P10 tissue disrupter (setting 10, 2 x 10 sec bursts). The homogenate is centrifuged at 48,000 x g for 12 min and the pellet obtained is washed, by resuspension and centrifugation, three times in homogenizing buffer. The tissue pellets are resuspended in the assay buffer, and are stored under liquid nitrogen until required.

The binding assays are conducted using a Tris-Krebs assay buffer of the following composition (mM):

NaCl, 154; KCl, 5.4; $KH_2PO_4$, 1.2; $CaCl_2.2H_2O$, 2.5; $MgCl_2$, 1.0; glucose, 11; Tris, 10. Assays are conducted at 25° C at 7.4 in a final volume of 0.25 ml. Zacopride (1.0 μM) is used to define the non-specific binding (NSB). 5-$HT_3$ receptors present in rat cortical membranes are labelled using 0.3-0.7 nM [$^3$H]quipazine (specific activity 50-66 Ci/mmol; New England Nuclear) in the presence of 0.1 μM paroxetine to prevent [$^3$H]quipazine binding to 5-HT uptake sites. The rat cortex membranes are incubated with [$^3$H]quipazine in the presence of 10 different concentrations of test compound ranging from $1x10^{-12}$ to $1x10^{-4}$ molar. Incubations are conducted for 45 min at 25° C and are terminated by vacuum filtration over Whatman GF/B glass fiber filters using a Brandel 48 well cell harvester. After filtration the filters are washed for 8 sec with 0.1 M NaCl. The filters are pretreated with 0.3% polyethyleneimine 18 hr prior to use in order to reduce filter binding of the radioligand. Radioactivity retained on the filters is determined by liquid scintillation counting.

The concentration of test compound producing 50% inhibition of radioligand binding is determined by an iterative curve fitting procedure. Affinities are expressed as the negative logarithm of the $IC_{50}$ value ($pIC_{50}$). Compounds of Formula I exhibit 5-$HT_3$ receptor binding affinity, i.e., $pIC_{50}$ values greater than 6.

EXAMPLE 8

5-$HT_3$ ANTAGONIST ACTIVITY IN RATS
(VON BEZOLD-JARISCH REFLEX)

The following describes an *in vivo* method for determining the 5-$HT_3$ antagonist activity of compounds of Formula I. The method is a modified version of that described by Butler et al., Cohen et al., and Fozard, all previously cited, in which the 5-$HT_3$ selective agonist 2-methyl-5-hydroxytryptamine (2-m-5-HT) is substituted for 5-HT.

Male Sprague-Dawley rats, 250-380 grams, are anesthetized with urethane (1.4 g/kg, i.p.). A tracheotomy is performed and a tube is inserted into the trachea to facilitate respiration. Jugular and femoral veins are canulated for intravenous administration of drug. The duodenum is canulated for intraduodenal administration of drug. Heart rate is monitored by Gould ECG/Biotech amplifiers. After at least a 30 min equilibration period and prior to administration of test compound, control responses to intravenous administration of 2-m-5-HT are determined and a minimal dose producing sufficient and consistent bradycardia is chosen.

Potency

Intravenous challenges to 2-m-5-HT are administered every 12 minutes. Either vehicle or test compound is administered intravenously 5 minutes before each challenge to 2-m-5-HT. Each successive administration of test compound is increased in dosage until responses to 2-m-5-HT are blocked.

Duration

Vehicle or test compound is administered intravenously or intraduodenally and subsequent challenges to 2-m-5-HT are administered at 5, 15, 30, 60, 120, 180, 240, 300 and, in some instances, 360, 420 and 480 minutes post dose.

For both potency and duration studies heart rate (beats/min) is recorded continuously for the duration of the study. Responses to 2-m-5-HT are represented by the peak decrease in heart rate. Effects of test compounds are represented as percent inhibition of the bradycardia induced by 2-m-5-HT. Data are analyzed by a one-way repeated measures ANOVA and followed by pairwise comparison to vehicle control using Fisher's LSD strategy. From a dose-response curve so constructed, an $ID_{50}$ value is obtained to represent the dose that inhibited 50% of the bradycardia induced by 2-m-5HT.

Compounds of Formula I exhibit 5-$HT_3$ receptor antagonist activity in this assay, i.e., $ID_{50}$ values less than 3.0 mg/kg, i.v.

EXAMPLE 9

CISPLATIN-INDUCED EMESIS IN FERRETS

The following describes the procedure for determining the intravenous (i.v.) effects of compounds of Formula I on cisplatin-induced emesis in ferrets.

Adult, male, castrated ferrets are allowed food and water ad libitum both prior to and throughout the testing period. Each animal is randomly chosen and anesthetized with a metofane/oxygen mixture, weighed and assigned to one of three test groups. While anesthetized an incision is made along the ventral cervical region approximately two to four centimeters in length. The jugular vein is then isolated and cannulated with a capped saline filled PE-50 polyethylene tubing. The cannula is exteriorized at the base of the skull and the incision closed with wound clips. The animals are then returned to their cages and allowed to recover from anesthesia prior to commencement of the study.

Vehicle or test compound is administered i.v. at 1.0 ml/kg and 1.0 mg/kg, respectively. Within 2.0 minutes of the administration of vehicle or test compound, cisplatin is injected i.v. at 10 mg/kg. The animals are then observed continuously for a 5 hour period and emetic responses (i.e., vomiting and/or retching) are recorded. For purposes of this example and that of Examples 10 and 11, vomiting is defined as the successful evacuation of stomach contents and a single episode of retching is defined as rapid and successive efforts to vomit occurring within a one minute time period.

Emetic responses are represented as (1) time to onset of emesis, (2) total vomiting episodes and (3) total retching episodes. Means and standard deviations of the test groups are compared to those of the reference groups. Significance is determined by Student's t-test when comparing a single treatment group to the vehicle control or by Dunnett's comparative analysis when more than one treatment group is compared to a single vehicle.

Intravenously administered compounds of Formula I are anti-emetic in this assay.

Proceeding as in Example 10 but administering the test compounds by oral route, the anti-emetic effects of compounds of Formula I may be evaluated. Orally administered compounds of Formula I are anti-emetic in this assay as shown by the following table:

| Treatment | Dose, P.O. (mg/kg) | N | Time to Onset | Retching Episodes | Vomiting Episodes |
|---|---|---|---|---|---|
| Vehicle | | 10 | 32.2±13.2 | 10.0±2.8 | 24.4±7.1 |
| (1) | | 4 | 42.8±44.9* | 4.0±2.45 | 13.0±9.4* |

(1) S-6-amino-2-(1-azabicyclo[2.2.2]oct-3-yl)-2,3-dihydro-1H-benz[de]-isoquinoline-1,3-dione hydrochloride

* $p < 0.05$, Student's T-Test

EXAMPLE 10

CISPLATIN-INDUCED EMESIS IN DOGS

The following describes the procedure for determining the intravenous (i.v.) effects of compounds of Formula I on cisplatin-induced emesis in dogs.

Male and female dogs (6-15 kg) are fed one cup of dry dog food. One hour following feeding, cisplatin (*cis*-diamminedichloroplatinum) is administered i.v. at 3 mg/kg. Sixty minutes after the administration of cisplatin, either vehicle or test compound is injected i.v. at 0.1 ml/kg and 1.0 mg/kg, respectively. The dogs are then observed continuously for a 5 hour period and the emetic responses (i.e., vomiting and/or retching) are recorded.

Emetic responses are represented as (1) time to onset of emesis, (2) total vomiting episodes and (3) total retching episodes. Means and standard deviations of the test groups are compared to those of the reference groups. Significance is determined by Student's t-test when comparing a single treatment group to the vehicle control or by Dunnett's comparative analysis when more than one treatment group is compared to a single vehicle.

Compounds of Formula I exhibit anti-emetic activity in this assay.

EXAMPLE 11

GASTRIC EMPTYING OF TEST MEAL IN RATS

The following describes an *in vivo* method of determining the prokinetic activity of the compounds of Formula I by measuring the rate of gastric emptying of test meal in rats. The method is that described by Droppleman et al., previously cited.

Test meal is prepared by slowly adding 20 grams of cellulose gum (Hercules Inc., Wilmington, Delaware) to 200 ml of cold distilled water that is being mixed in a Waring blender at approximately 20,000 rpm. Mixing continues until complete dispersion and hydration of the cellulose gum takes place (approximately 5 min). Three beef bouillon cubes are dissolved in 100 ml of warm water and then blended into the cellulose solution followed by 16 g of purified casein (Sigma Chemical Co., St. Louis, MO), 8 g of powdered confectioners sugar, 8 g of cornstarch, and 1 g of powdered charcoal. Each ingredient is added slowly and mixed thoroughly resulting in approximately 325 ml of a dark gray to black, homogenous paste. The meal is then refrigerated overnight during which time trapped air escapes. Prior to the assay the meal is removed from the refrigerator and allowed to warm to room temperature.

Mature (170 to 204 g) male Sprague-Dawley rats are deprived of food for 24 hours with water ad libitum. On the morning of the study each animal is weighed and randomly assigned to treatment groups consisting of ten animals per group. Each rat receives either vehicle, test compound or the reference standard metoclopramide by intraperitoneal injection. At 0.5 hours post injection 3.0 ml of test meal is orally administered to each rat with a 5.0 ml disposable syringe. Five test meal samples are weighed on an analytical balance and these weights are averaged to find a mean test meal weight. At 1.5 hours post injection each rat is sacrificed by carbon dioxide asphyxiation and the stomach is removed by opening the abdomen and carefully clamping and cutting the esophagus just below the pyloric sphincter. Taking care not to lose any of the its contents, each stomach is placed on a small, pre-weighed and correspondingly labeled 7 ml weigh boat and immediately weighed on an analytical balance. Each stomach is then cut open along the lesser curvature, rinsed with tap water, gently blotted dry to remove excess moisture and weighed. The amount of test meal remaining in the stomach is represented by the difference between the weight of the full stomach and the weight of the stomach empty. The difference between the amount of test meal remaining and the mean test meal weight represents the quantity of test meal that empties during the 1.5 hour post injection period.

Responses are represented as grams of meal emptied or percent change from control. Means and standard deviations of the test groups are compared to those of the reference groups. Significance is determined via Dunnett's t-test (Statistical Association Journal, December 1955, 1096-112).

Compounds of Formula I exhibit prokinetic activity in this assay.

EXAMPLE 12

THE MOUSE ANXIOLYTIC BEHAVIOR MODEL

The following describes an *in vivo* method for determining anxiolytic activity of compounds of Formula I.

Naive male C5Bl/6J mice, 18-20 g, are kept in groups of 10 mice in quarters controlled for sound, temperature and humidity. Food and water are available ad libitum. The mice are kept on a 12 hour light and 12 hour dark cycle, with lights on at 6:00 a.m. and off at 6:00 p.m. All experiments begin at least 7 days after arrival on site.

The automated apparatus for detecting changes in exploration is obtained from Omni-Tech Electronics Columbus Ohio and is similar to that of Crawley and Goodwin (1980), as described in Kilfoil et al., cited previously. Briefly, the chamber consists of a plexiglass box (44 x 21 x 21 cm), divided into two chambers by a black plexiglass partition. The partition dividing the two chambers contains a 13 x 5 cm opening through which the mouse can easily pass. The dark chamber has clear sides and a white floor. A fluorescent tube light (40 watt) placed above the chambers provides the only illumination. The Digiscan Animal Activity Monitor System RXYZCM16 (Omni-Tech Electronics) records the exploratory activity of the mice within the test chambers.

Prior to commencement of the study the mice are given 60 min to acclimatize to the laboratory environment. After a mouse receives an intraperitoneal (i.p.) injection of either test compound or vehicle it is returned to its home cage for a 15 min post-treatment period. The mouse is then placed in the center of the light chamber and monitored for 10 minutes.

Anxiolysis is seen as a general increase in exploratory activity in the lighted area. An increase in exploratory activity is relected by increased latency (the time for the mouse to move to the dark chamber when first placed in the center of the lighted area), increase in shuttle activity, increased or unaltered locomotor activity (number of grid lines crossed) and decreased time spent in the dark compartment.

Compounds of Formula I exhibit anxiolytic activity in this assay as shown by the following table:

| COMPOUND | SHUTTLE ACTIVITY | | LOCOMOTOR ACTIVITY (LIGHT AREA) | | TIME DARK AREA (SEC) | | LATENCY (SEC) | | % ACTIVITY DARK AREA 2* | |
|---|---|---|---|---|---|---|---|---|---|---|
| | Mean ± SD | W% | Mean ± SD | W% | Mean ± SD | W% | Mean ± SD | W% | Mean ± SD | W% |
| Ondansetron | 121.0 ± 12.4* | 182.7 | 1582.0 ± 77.8* | 52.6 | 499.4 ± 14.8* | -12.8 | 6.7 ± 1.5 | 4.7 | 78.5 ± 4.7 | -13.1 |
| (1) | 42.0 ± 3.9 | 37.3 | 1208.0 ± 86.4 | 41.0 | 394.9 ± 32.8 | -26.9 | 3.5 ± 0.5 | -28.6 | 37.6 ± 4.9* | -54.0 |
| (2) | 138.6 ± 9.1* | 112.3 | 1602.4 ± 73.0 | 42.6 | 498.4 ± 9.3 | -10.8 | 8.1 ± 1.4 | 102.5 | 78.4 ± 1.8* | -10.0 |

* Significance from control; $p < 0.05$

Percentage change from control ( % ) is calculated and statistical analysis is performed using controls particular to each experimental run.

2* Unaltered or decreased % locomotor activity in the dark area is an indication that increased exploratory activity is not the results of an overall increase in locomotor activity but is the reflection of anxiolytic activity.

(1) S-6-amino-5-chloro-2-(1-azabicyclo[2.2.2]oct-3-yl)-2,3- dihydro-1H-benz[de]isoquinoline-1,3-dione hydrochloride.

(2) S-6-amino-5-chloro-2-(1-azabicyclo[2.2.2]oct-3-yl)-2,3- dihydro-1H-benz[de]isoquinoline-1,3-dione hydrogeniodide.

EXAMPLE 13

The Mouse Light/Dark Withdrawal Anxiety Test

The following procedure describes a method to determine whether compounds of Formula I effect the anxiety that occurs after abruptly ceasing chronic treatment with drugs of abuse.

Naive male BKW mice (25-30 g) are caged in groups of ten in quarters controlled for sound, temperature and humidity. Food and water are available ad libitum. The mice are kept on a 12 hour light cycle and 12 hour dark cycle, with lights on at 6:00 a.m. and off at 6:00 p.m. All experiments begin at least 7 days after arrival on site.

Levels of anxiety are determined by the two-compartment exploratory model of Crawley and Goodwin (see Example 12). Anxiolysis is seen as a general increase in exploratory activity in the lighted area. An increase in exploratory activity is relected by increased latency (the time for the mouse to move to the dark chamber when first placed in the center of the lighted area), increased or unaltered locomotor activity (number of grid lines crossed), increased number of rears and decreased time spent in the dark compartment.

Increased exploratory activity in the lighted area is induced by treating the mice for 14 days with alcohol (8.0 % w/v in drinking water), nicotine (0.1 mg/kg, i.p., twice daily), diazepam (10 mg/kg, i.p., twice daily), or cocaine (1.0 mg/kg, i.p., twice daily). Anxiolysis is assessed 1, 3, 7 and 14 days after commencement of the drug regime. The treatment is abruptly ceased and exploratory activity in the lighted area is determined 8, 24 and 48 hours thereafter. Vehicle or test compounds are administered during the withdrawl phase by intraperitoneal injection. Responses are represented as inhibition of the decrease in anxiolytic behavior after the alcohol, cocaine or nicotine treatment is ceased.

Intraperitoneal adminitration of compounds of Formula I decrease the anxiety associated with drug withdrawal in this model as shown by the following table:

| Treatment | Time in Dark (%) | Latency (sec.) | Rears/5 Min in Light | Crossings/ 5 Min in Light |
|---|---|---|---|---|
| Control | 58.3±5.9 | 8.0±0.7 | 22.4±2.4 | 24.4±2.7 |
| diaz W/D | 70.0±8.0 | 1.8±0.1 | 8.4±0.9 | 7.8±0.9 |
| W/D+(1)° | 27.4±2.9 | 34.0±3.7 | 89.2±9.1 | 97.5±10.6 |
| Control | 59.0±6.0 | 9.6±1.5 | 26.0±2.8 | 33.0±3.6 |
| nic W/D | 69.7±7.0 | 2.0±0.01 | 9.6±1.1 | 10.4±1.4 |
| W/D+(1)° | 29.0±3.1 | 20.8±2.4 | 84.5±9.0 | 94.0±10.1 |
| Control | 58.4±6.0 | 7.3±0.9 | 28.6±3.2 | 37.0±4.0 |
| alc W/D | 80.0±8.2 | 2.0±0.3 | 12.3±1.8 | 14.3±1.7 |
| W/D+(1)° | 60.0±6.4 | 9.0±1.3 | 86.0±9.0 | 88.0±9.0 |
| Control | 58.0±5.9 | 9.8±1.5 | 30.2±3.3 | 34.2±3.6 |
| coc W/D | 74.5±7.50 | 1.8±0.2 | 8.6±1.0 | 8.0±0.9 |
| W/D+(1)° | 22.5±2.4 | 28.7±2.81 | 101.±2.81 | 135.±15.7 |

W/D = withdrawal
diaz = diazepam; nic = nicotine; alc = alcohol; coc = cocaine
* significance from control; $p < 0.01$
‡ significance from W/D; $p < 0.01$
° 1 µ/g/kg of C(HCl) is administered i.p.
(1) S-6-amino-5-chloro-2-(1-azabicyclo[2.2.2]oct-3-yl)-2,3-dihydro-1H-benz[de]isoquinoline-1,3-dione hydrochloride

EXAMPLE 14

The Mouse Habituation/Cognitive Enhancement Test

The following describes a model to determine the cognitive enhancing effects of compounds of Formula I.

Young adult and aged BKW mice are caged in groups of ten in quarters controlled for sound, temperature and humidity. Food and water are available ad libitum. The mice are kept on a 12 hour light cycle and 12 hour dark cycle, with lights on at 6:00 a.m. and off at 6:00 p.m. All experiments begin at least 7 days after arrival on site.

Levels of anxiety are determined by the two-compartment exploratory model of Crawley and Goodwin (see Example 12). Mice are exposed to the two-compartment test area over a 3 day period. The young mice habituate to the test area by day 3 and spend less time exploring the lighted area, whereas exploratory activity in the lighted area remains constant through day 3 for the aged mice. Exploratory activity is seen as latency (the time for the mouse to move to the dark chamber when first placed in the center of the lighted area), locomotor activity (number of grid lines crossed), number of rears and time spent in the lighted compartment. Vehicle or test compounds are administered to the aged mice by intraperitoneal injection. Cognitive enhancing effects in the aged rats are reflected by a decrease in

exploratory activity by day 3.

Intraperitoneal adminitration of compounds of Formula I enhance cognition in this model as shown by the following results:

| | Time Dark Area (%)[1] | Day 3 Latency (sec.)[2] | Rears[3] | Locomotor Activity[4] |
|---|---|---|---|---|
| young (control) | 82.9±8.9 | 5.4±1.2 | 16.2±1.5 | 23.6±2.3 |
| aged (control) | 32.9±3.1 | 36.3±4.6 | 49.2±5.4 | 60.0±6.9 |
| aged treated [5] | 59.7±5.4* | 5.0±0.4* | 13.1±1.5* | 20.0±1.9* |

1 Percentage of time over a minute period spent in the dark chamber.

2 The time for the mouse to move to the dark chamber when first placed in the center of the lighted chamber.

3 Number of rears/5 min. in the lighted chamber.

4 Number of grid crossings/5 min. in the lighted chamber.

5 S-6-amino-5-chloro-2-(1-azabicyclo[2.2.2]oct-3-yl)-2,3-dihydro-1H-benz[de]isoquinoline-1,3-dione hydrochloride.

* Improved habituation compared with aged controls.

EXAMPLE 15

The following are representative pharmaceutical formulations containing a compound of Formula I.

## ORAL FORMULATION

A representative solution for oral administration contains:

| | |
|---|---|
| A compound of Formula I* | 100-1000 mg |
| Citric Acid Mono hydrate | 105 mg |
| Sodium Hydroxide | 18 mg |
| Flavouring | q.s. |
| Water | to 100 ml |

## INTRAVENOUS FORMULATION

A representative solution for intravenous administration contains:

| | |
|---|---|
| A compound of Formula I* | 10-100 mg |
| Dextrose Monohydrate | q.s to make isotonic |
| Citric Acid Monohydrate | 1.05 mg |
| Sodium Hydroxide | 0.18 mg |
| Water for Injection | to 1.0 ml |

*Example: S- or RS-2-(1-azabicyclo[2.2.2]oct-3-yl)-2,3-dihydro-1H-benz[de]isoquinoline-1,3-dione hydrochloride or S-2-(1-azabicyclo[2.2.2]oct-3-yl)-2,3-dihydro-1H-benz[de]isoquinoline-3-one hydrochloride, or S-6-amino-5-chloro-2-(1-azabicyclo[2.2.2]oct-3-yl)-2,3-dihydro-1H-benz[de]isoquinoline-1,3-dione hydrochloride.

EXAMPLE 16
(Toxicity of the Compounds of Formula I)

The following compounds were administered at 10 mg/kg, i.p. No unusual effects were observed over 90 minutes:

S- and RS-6-chloro-5-amino-2-(1-azabicyclo[2.2.2] oct-3-yl)-2,3-dihydro-1H-benz[de]isoquinoline-1,3-dione hydrochloride and S-2-(1-azabicyclo[2.2.2]oct-3-yl)-2,3-dihydro-1H-benz[de]isoquinoline-1,3-dione hydrochloride.

EXAMPLE 17

The following is an example of Scheme I in the synthesis of R-2-(1-azabicyclo[2.2.2]oct-3-yl)-2,3-dihydro-1H-benz[de]isoquinoline-1,3-dione, a compound of the Formula I wherein Z is an oxo group, X and Y are hydrogen and $R^1$ is chosen from subformula (b) wherein n is 2, p is zero and the $R^1$ substituent through its 3-position is attached to the nitrogen of the isoquinoline ring.

A. 3.15 Grams of 1,8-naphthalic anhydride are heated to 140°C in about 150 ml of xylenes until the anhydride is in solution. 2 Grams of R-3-aminoquinuclidine in 80 to 100 ml xylenes are added and the resulting mixture is refluxed overnight. The mixture is filtered, concentrated and chromatographed (3.5 cm x 19 cm silica-gel), eluting with a dichloromethane/5% methanol mixture containing ammonia. 3.3 Grams of R-2-(1-azabicyclo[2.2.2]oct-3-yl-2,3-dihydro-1H-benz[de]isoquinoline-1,3-dione of a melting point of 187-91°C and $[\alpha]_D$ +80° [.415 chloroform] are obtained.

In a similar manner the S-isomer is prepared with a melting point of 185-9°C and an $[\alpha]_D$ -73.8° [.535 chloroform].

B. 300 Milligrams of the R-isomer are dissolved in 6 ml ethanol and treated with a stoichiometric excess of hydrochloric acid to yield 300 mgs of hydrochloride salt with a melting point of 325-6°C and an $[\alpha]_D$ +26.5° [.535 chloroform]. Anal.: Calcd. for $C_{19}H_{19}N_2O_2Cl$. 0.5 $H_2O$ (MW 351.84): C, 64.86; H, 5.73; N, 7.96. Found: C, 65.00; H, 5.93; N, 7.64.

EXAMPLE 18

The following is an example of the reduction of a compound of the Formula I wherein Z represents an oxo group to prepare a compound of Formula I wherein Z represents two hydrogen atoms. Specifically the reduction described involves the R-isomer of Part A of Example 17 as starting material, resulting in the synthesis of a compound of the Formula I wherein Z represents two hydrogen atoms, X and Y are hydrogen and $R^1$ is chosen subformula (b) wherein n is two, p is zero and the $R^1$ substituent through its 3-position is attached to the nitrogen atom of the isoquinoline ring.

A. 2.5 Grams of sodium boranate are dissolved in 100 ml ethanol and 20 ml water. The solution is cooled in an ice-water bath and 2.1 grams of R-2-(1-azabicyclo[2.2.2]oct-3-yl-2,3-dihydro-1H-benz[de]-isoquinoline-1,3-dione of Part A of Example 17 in 100 ml of ethanol is added dropwise at a rapid rate over 15 minutes. The reaction mixture is kept at room temperature for 5 hours, is concentrated and water is added. The mixture is stirred while cooled in an ice-bath. Concentrated hydrochloric acid is added until the solution remains acidic, and then stirred over night. The mixture is filtered, basified with sodium hydroxide and extracted with dichloromethane. The extract is subjected to TLC in 10% methanol in dichloromethane and ammonia. The dicloromethane extract is concentrated, and dried to give 1.5 grams of R-2-(1-azabicyclo[2.2.2]oct-3-yl-2,3-dihydro-1H-benz[de]isoquinoline-3-one. This compound is dissolved in 30 ml acetone, and under stirring 200 milligrams of hydrochloric acid in 2 ml of ethanol are added. After the addition is complete very sudden crystallization of the product takes place. 1.24 Grams of the product are obtained with a melting point of 270-85° C. Because the product turned brown gradually beginning at about 220° C, it was recrystallised from 20 ml ethanol to give 580 milligrams of the R-isomer of a melting point of 279-81° C. Anal.: Calcd. for $C_{19}H_{21}N_2OCl$. (MW 328.84): C, 69.39; H, 6.43; N, 8.51. Found: C, 69.29; H, 6.59; N, 8.57 $[\alpha]_D$-12.3° (MeOH).

EXAMPLE 19

The following is an example of the synthesis of a compound of Formula I wherein Z is an oxo group, X is chloro in the 6-position, Y is hydrogen and $R^1$ is chosen from subformula (b) wherein n is two and p is zero and the $R^1$ substituent through its 3-position is attached to the nitrogen atom of the isoquinoline ring, i.e. S-6-chloro-2-(1-azabicyclo[2.2.2]oct-3-yl)-2,3-dihydro-1H -benz[de]isoquinoline-1,3-dione.

1 Gram of 4-chloro-1,8-naphthalic anhydride and 550 milligrams of S-3-aminoquinuclidine in 75 ml of ethanol are kept at reflux overnight. One spoonful of charcoal is added to the mixture. The TLC is run in 5% methanol in dichloromethane with ammonia. The filtrate is concentrated to dryness and chromatographed through 2.3 cm x 14 cm of silicagel, followed by elution with 500 ml of 3% methanol in dichloromethane and then 5% methanol. O.7 Grams of a solid are obtained which was heated with 10 ml of acetone, and cooled to room temperature. This procedure removes some yellowish color and leaves 0.7 grams of the product off-white with a melting point of 235-8° C. The product should be kept in the dark, as it is somewhat light sensitive. $[\alpha]_D$-85.5° [.51 dichloromethane].

The base is dissolved in 15 ml of hot ethanol, and 100 milligrams of hydrochloric acid in 1 ml of ethanol is added to form the hydrochloride; melting point 348° C; $[\alpha]_D$-29.95° [1.075 water].

EXAMPLE 20

The following is an example of Scheme II Alt. 2 and involves the halogenation of a compound of Formula I wherein Z is the oxo group and X and Y are both hydrogen and the $R^1$ substituent is chosen from subformula (a) wherein n is 2, p is zero and $R^2$ is methyl and the $R^1$ substituent through its 3-position is attached to the nitrogen atom of the isoquinoline ring. Specifically, this example prepares 6-amino-5-chloro-2-(endo-8-methyl-8-azabicyclo[3.2.1]oct-3-yl)-2,3-dihydro-1H-benz[de]isoquinoline-1,3-dione hydrochloride.

580 Milligrams of 6-amino-2-(endo-8-methyl-8-azabicyclo[3.2.1]oct-3-yl)-2,3-dihydro-1H-benz[de]-isoquinoline-1,3-dione hydrochloride with 15 ml of dimethylformamide are stirred at room temperature and 230 milligrams of N-chlorosuccinimide are added in small portions. After 1 and 4 hours the TLC shows the desired chlorinated product but also unreacted starting material. Therefore an additional 100 mg of N-chlorosuccinimide are added. Two hours later the TLC shows mostly the reaction product but still some minor amount of starting material. The reaction mixture is stirred overnight, after which time the TLC shows an essentially completed reaction. The reaction mixture is poured into 250 ml of stirred ethylacetate, the solid formed is collected and treated with boiling ethanol, cooled to room temperature, collected again and dried in a vacuum at 78C. 380 Milligrams are obtained with a melting point of 306-9° C. Anal.: Calcd. for $C_{20}H_{21}N_3O_2Cl_2.0_5.H_2O$ (MW 415.32): C, 57.83; H, 5.33; N, 10.12. Found: C, 57.67; H, 5.64; N, 9.82.

EXAMPLE 21

The following is an example of Alt. 4 of Scheme II, showing the displacement of a nitro group with a lower alkylamino group, resulting in the preparation of a compound of Formula I wherein Z is an oxo group, X is an n-butylamino group in the 6-position and $R^1$ is chosen from subformula (b) wherein n is two and p is zero and the $R^1$ substituent through its 3-position is attached to the nitrogen of the isoquinoline ring.

To 400 milligrams of S-6-nitro-2-(1-azabicyclo[2.2.2]oct-3-yl)-2,3-dihydro-1H-benz[de]-isoquinoline-1,3 -dione in 10 ml DMF are added 40 drops of n-butylamine. The reaction mixture is kept over night at room temperature. On the next day, the TLC still shows some unreacted material but also product. The mixture is stirred at room temperature for an additional 60 hours. Thereafter the reaction mixture is kept at 50 °C on a rotatory evaporator for at least 30 minutes to remove as much of the n-butylamine as possible. A small amount of ethanolic hydrochloric acid (100 mg) is added and the entire mixture poured into 150 ml of stirred ether. A precipitate is formed which sticks to the wall of the reaction vessel. The solvent is decanted, and the precipitate rinsed with fresh ether. The material is chromatographed, yielding 200 milligrams of the hydrochloride salt of S-6-n-butylamino-2-(1-azabicyclo[2.2.2]oct-3-yl)-2,3-dihydro-1H-benz[de]-isoquinoline-1,3 -dione. The salt, in turn, is dissolved in a few mls of acetone and a little ethanolic hydrochloric acid added. The precipitated salt is collected and dried in a vacuum (100 mg).

Anal.: Calcd. for $C_{23}H_{28}N_3O_2Cl$ (MW 413.95): C, 66.73; H, 6.82; N, 10.15. Found: C, 66.96; H, 7.10; N, 9.97.

EXAMPLE 22

The following is an example of Alt. 3 of Reaction Scheme II, followed by a variation of Alt. 2 of Scheme II in which instead of the amino-substituted compound an acylamino-substituted compound is being used as the starting material. This results in the preparation of a compound of the Formula I wherein Z is an oxo group, X is a 6-acetylamino group and $R^1$ is chosen from subformula (b) wherein n is two, p is zero and $R^1$ through its 3-position is attached to the nitrogen of the isoquinoline ring. This is followed by the preparation of the corresponding 5-chloro-6-acetylamino compound.

2.27 Grams of S-6-amino-2-(1-azabicyclo[2.2.2]oct-3-yl)-2,3- dihydro-1H-benz[de]-isoquinoline-1,3-dione dissolved in 25 ml of acetic acid are kept at 100-10 °C (oilbath) and 12 ml acetic anhydride are added. After one hour the acylation is complete. The reaction mixture is concentrated to a small volume and squirted into 100 ml of well stirred ether. The mixture is stirred rapidly until it has a fine powdery appearance. 2.44 grams of S-6-acetylamino-2-(1-azabicyclo [2.2.2]oct-3-yl)-2,3-dihydro-1H-benz[de]isoquinoline-1,3-dione are collected and dried over night under reflux ethanol heating. The compound is converted to its hydrochloride salt.

0.7 grams of the acetanilide hydrochloride and 0.25 grams of N-chlorosuccinimide are dissolved in 25 to 30 ml of DMF. Additional N-chlorosuccinimide is added and the mixture is stirred over night. All is in solution and the reaction is complete. Most of the DMF is removed on an oil-pump, ethyl acetate is added to the remaining viscous oil. The flask is scratched until some solidification occurs. The stirring is continued until all appears powdery. The solid is collected and dissolved in about 50 ml of hot ethanol. Upon cooling to room temperature no crystallization occurs but dropwise addition of ether almost instantaneously gives precipitation of a gum. The solvent is decanted. Standing for two days gives a yellowish sticky layer coating the walls of the flask and some white crystals. The solvent is decanted again and seeded with the white crystals. After standing over 48 hours 0.14 grams of S-6-acetylamido-5-chloro-2-(1-azabicyclo[2.2.2]oct-3-yl)-2,3-dihydro-1H-benz[de]-isoquinoline-1,3-dione are isolated as the hydrochloride salt with gradual melting at > 205 °C, $[\alpha]_D$ [$H_2$0 .00555] -32.8 °.

EXAMPLE 23

This example describes the oxidation of a compound of the Formula I wherein X is chloro in the 6-position, Y is amino in the 5-position, Z is an oxo group and $R^1$ is chosen from subformula (b) wherein n is two and p is zero and the $R^1$ substituent through its 3-position is attached to the nitrogen atom of the quinoline ring to the corresponding N-oxide wherein p is one, i.e., the compound S-6-chloro-5-amino-2-(1-azabicyclo[2.2.2] oct-3-yl)-2,3-dihydro-1H- benz[de]isoquinoline-1,3-dione N-oxide.

560 Milligrams of the free base are dissolved in 30 mls methanol with 30 mls chloroform and 1 ml of 30% hydrogen peroxide is added. The reaction mixture is stirred overnight, after which a test for the presence of the N-oxide was positive. An additional 0.5 ml of 30% hydrogen perioxide is added and the mixture stirred over the weekend. Thereafter an additional 1 ml of 30% hydrogen peroxide is added. The

reaction mixture was warmed to 65 °C and gently boiled under reflux. After standing overnight, 0.6 ml of 30% hydrogen peroxide was added and the mixture refluxed again. 300 Milligrams of the N-oxide were obtained of a melting point of > 200 °C with foaming at 245 °C.

## Claims

**1.** A compound of Formula I

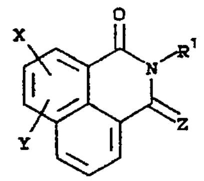

I

in which

Z is an oxo group or two hydrogens;

X and Y are independently selected from hydrogen, halogen, hydroxy, lower alkoxy, benzyloxy, lower alkyl, lower cycloalkyl, nitro, amino, amino-carbonyl, (lower alkyl)amino, di(lower alkyl)amino and (lower alkanoyl)amino;

$R^1$ is selected from

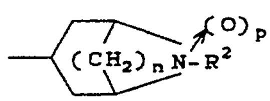

(a)

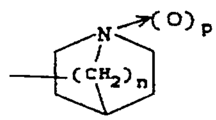

(b)

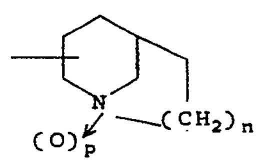

(c)

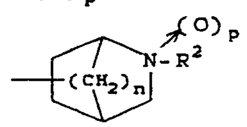

(d)

in which

p is 0 or 1;

n is 1, 2 or 3; and

$R^2$ is hydrogen, lower alkyl, $C_{3-8}$ cycloalkyl, $C_{3-8}$ cycloalkyl-$C_{1-2}$ alkyl, or a group $(CH_2)_tR^3$ where t is 1 or 2 and $R^3$ is thienyl, pyrrolyl or furyl optionally further substituted by one or two substituents selected from lower alkyl, lower alkoxy, trifluoromethyl or halogen, or is phenyl optionally substituted by one or two substituents selected from $C_{1-4}$ alkoxy, trifluoromethyl, halogen, nitro, carboxy, esterified carboxy, and $C_{1-4}$ alkyl optionally substituted by hydroxy, $C_{1-4}$ alkoxy, carboxy, esterified carboxy or in vivo hydrolyzable acyloxy; or

a pharmaceutically acceptable salt thereof, or an individual isomer or mixture of isomers thereof.

2. The compound of Claim 1 wherein
X and Y are independently selected from hydrogen, halogen, amino, nitro, amino-carbonyl or lower alkylamino, p is zero; and, in case $R^1$ comprises a bicyclic substituent with $R^2$ such $R^2$ is lower alkyl.

3. The compound of Claim 2 wherein Z is oxygen and $R^2$ is methyl.

4. The compound of Claim 3 wherein $R^1$ is
1-azabicyclo[2.2.2]oct-3-yl;
1-azabicyclo[2.2.2]oct-4-yl;
9-methyl-9-azabicyclo[3.3.1]non-3-yl;
8-methyl-8-azabicyclo[3.2.1]oct-3-yl; or
1-azabicyclo[3.3.1]non-4-yl.

5. A compound of Claim 4 selected from:
6-amino-5-chloro-2-(1-azabicyclo[2.2.2]oct-3-yl)-2,3-dihydro-1*H*-benz[*de*]isoquinoline-1,3-dione or a pharmaceutically acceptable salt thereof;
*S*-6-amino-5-chloro-2-(1-azabicyclo[2.2.2]oct-3-yl)-2,3-dihydro-1*H*-benz[*de*]isoquinoline-1,3-dione or a pharmaceutically acceptable salt thereof;
*S*-6-amino-5-chloro-2-(1-azabicyclo[2.2.2]oct-3-yl)-2,3-dihydro-1*H*-benz[*de*]isoquinoline-1,3-dione hydrochloride;
*R*-6-amino-5-chloro-2-(1-azabicyclo[2.2.2]oct-3-yl)-R,3-dihydro-1*H*-benz[*de*]isoquinoline-1,3-dione or a pharmaceutically acceptable salt thereof; and
*R*-6-amino-5-chloro-2-(1-azabicyclo[2.2.2]oct-3-yl)-2,3-dihydro-1*H*-benz[*de*]isoquinoline-1,3-dione hydrochloride.

6. A compound of Claim 4 selected from:
6-amino-5-iodo-2-(1-azabicyclo[2.2.2]oct-3-yl)-2,3-dihydro-1*H*-benz[*de*]isoquinoline-1,3-dione or a pharmaceutically acceptable salt thereof;
*S*-6-amino-5-iodo-2-(1-azabicyclo[2.2.2]oct-3-yl)-2,3-dihydro-1*H*-benz[*de*]isoquinoline-1,3-dione or a pharmaceutically acceptable salt thereof;
*S*-6-amino-5-iodo-2-(1-azabicyclo[2.2.2]oct-3-yl)-2,3-dihydro-1*H*-benz[*de*]isoquinoline-1,3-dione hydrochloride;
*R*-6-amino-5-iodo-2-(1-azabicyclo[2.2.2]oct-3-yl)-2,3-dihydro-1*H*-benz[*de*]isoquinoline-1,3-dione or a pharmaceutically acceptable salt thereof; and
*R*-6-amino-5-iodo-2-(1-azabicyclo[2.2.2]oct-3-yl)-2,3-dihydro-1*H*-benz[*de*]isoquinoline-1,3-dione hydrochloride.

7. A compound of Claim 4 selected from:
6-amino-5-nitro-2-(1-azabicyclo[2.2.2]oct-3-yl)-2,3-dihydro-1*H*-benz[*de*]isoquinoline-1,3-dione or a pharmaceutically acceptable salt thereof;
*S*-6-amino-5-nitro-2-(1-azabicyclo[2.2.2]oct-3-yl)-2,3-dihydro-1*H*-benz[*de*]isoquinoline-1,3-dione or a pharmaceutically acceptable salt thereof;
*S*-6-amino-5-nitro-2-(1-azabicyclo[2.2.2]oct-3-yl)-2,3-dihydro-1*H*-benz[*de*]isoquinoline-1,3-dione hydrochloride;
*R*-6-amino-5-nitro-2-(1-azabicyclo[2.2.2]oct-3-yl)-2,3-dihydro-1*H*-benz[*de*]isoquinoline-1,3-dione or a pharmaceutically acceptable salt thereof; and
*R*-6-amino-5-nitro-2-(1-azabicyclo[2.2.2]oct-3-yl)-R,3-dihydro-1*H*-benz[*de*]isoquinoline-1,3-dione hydrochloride.

8. A compound of Claim 4 selected from:
6-amino-2-(1-azabicyclo[2.2.2]oct-3-yl)-2,3-dihydro-1*H*-benz[*de*]isoquinoline-1,3-dione or a pharmaceutically acceptable salt thereof;
*S*-6-amino-2-(1-azabicyclo[2.2.2]oct-3-yl)-2,3-dihydro-1*H*-benz[*de*]isoquinoline-1,3-dione or a pharmaceutically acceptable salt thereof;
*S*-6-amino-2-(1-azabicyclo[2.2.2]oct-3-yl)-2,3-dihydro-1*H*-benz[*de*]isoquinoline-1,3-dione hydrochloride;
*R*-6-amino-2-(1-azabicyclo[2.2.2]oct-3-yl)-2,3-dihydro-1*H*-benz[*de*]isoquinoline-1,3-dione or a pharmaceutically acceptable salt thereof; and
*R*-6-amino-2-(1-azabicyclo[2.2.2]oct-3-yl)-2,3-dihydro-1*H*-benz[*de*]isoquinoline-1,3-dione hydrochloride.

9. A compound of Claim 4 selected from:
6-amino-5-chloro-2-(9-methyl-9-azabicyclo[3.3.1]non-3-yl) -2,3-dihydro-1*H*-benz[*de*]isoquinoline-1,3-dione or a pharmaceutically acceptable salt thereof;
6-amino-5-chloro-2-(*endo*-9-methyl-9-azabicyclo[3.3.1]non-3-yl)-2,3-dihydro-1*H*-benz[*de*]isoquinoline-1,3-dione or a pharmaceutically acceptable salt thereof;
6-amino-5-chloro-2-(*endo*-9-methyl-9-azabicyclo[3.3.1]non-3-yl)-2,3-dihydro-1*H*-benz[*de*]isoquinoline-1,3-dione hydrochloride;
6-amino-5-chloro-2-(*exo*-9-methyl-9-azabicyclo[3.3.1]non-3-yl)-2,3-dihydro-1*H*-benz[*de*]isoquinoline-1,3-dione or a pharmaceutically acceptable salt thereof; and
6-amino-5-chloro-2-(*exo*-9-methyl-9-azabicyclo[3.3.1]non-3-yl)-2,3-dihydro-1*H*-benz[*de*]isoquinoline-1,3-dione hydrochloride.

10. A compound of Claim 4 selected from:
6-amino-5-chloro-2-(8-methyl-8-azabicyclo[3.2.1]oct-3-yl) -2,3-dihydro-1*H*-benz[*de*]isoquinoline-1,3-dione or a pharmaceutically acceptable salt thereof;
6-amino-5-chloro-2-(*endo*-8-methyl-8-azabicyclo[3.2.1]oct-3-yl)-2,3-dihydro-1*H*-benz[*de*]isoquinoline-1,3-dione or a pharmaceutically acceptable salt thereof;
6-amino-5-chloro-2-(*endo*-8-methyl-8-azabicyclo[3.2.1]oct-3-yl)-2,3-dihydro-1*H*-benz[*de*]isoquinoline-1,3-dione hydrochloride;
6-amino-5-chloro-2-(*exo*-8-methyl-8-azabicyclo[3.2.1]oct-3-yl)-2,3-dihydro-1*H*-benz[*de*]isoquinoline-1,3-dione or a pharmaceutically acceptable salt thereof; and
6-amino-5-chloro-2-(*exo*-8-methyl-8-azabicyclo[3.2.1]oct-3-yl)-2,3-dihydro-1*H*-benz[*de*]isoquinoline-1,3-dione hydrochloride.

11. A compound of Claim 4 selected from:
6-amino-5-chloro-2-(1-azabicyclo[3.3.1]non-4-yl)-2,3-dihydro-1*H*-benz[*de*]isoquinoline-1,3-dione or a pharmaceutically acceptable salt thereof;
6-amino-5-chloro-2-(*endo*-1-azabicyclo[3.3.1]non-4-yl)-2,3 -dihydro-1*H*-benz[*de*]isoquinoline-1,3-dione or a pharmaceutically acceptable salt thereof;
6-amino-5-chloro-2-(*endo*-1-azabicyclo[3.3.1]non-4-yl)-2,3 -dihydro-1*H*-benz[*de*]isoquinoline-1,3-dione hydrochloride;
6-amino-5-chloro-2-(*exo*-1-azabicyclo[3.3.1]non-4-yl)-2,3-dihydro-1*H*-benz[*de*]isoquinoline-1,3-dione or a pharmaceutically acceptable salt thereof; and
6-amino-5-chloro-2-(exo-1-azabicyclo[3.3.1]non-4-yl)-2,3-dihydro-1*H*-benz[*de*]isoquinoline-1,3-dione hydrochloride.

12. A compound of Claim 4 selected from:
6-amino-5-chloro-2-(1-azabicyclo[2.2.2]oct-4-yl)-2,3-dihydro-1*H*-benz[*de*]isoquinoline-1,3-dione or a pharmaceutically acceptable salt thereof; and
6-amino-5-chloro-2-(1-azabicyclo[2.2.2]oct-4-yl)-2,3-dihydro-1*H*-benz[*de*]isoquinoline-1,3-dione hydrochloride.

13. A compound of Claim 1 wherein p is 1 and
X and Y are independently selected from hydrogen, halogen, amino, nitro and amino-carbonyl, or lower alkylamino; and in case $R^1$ comprises a bicyclic substituent with $R^2$ such $R^2$ is lower alkyl.

14. The compound of Claim 13 wherein Z is oxygen and $R^2$ is methyl.

15. A compound according to any of Claims 1 to 14 for use as a pharmaceutical.

16. The use of a compound according to any of Claims 1 to 14 in the preparation of a medicament for the treatment of emesis, nausea, a gastro-intestinal disorder, migraine, a CNS disorder, a cardiovascular disorder or pain.

17. A pharmaceutical composition comprising a compound of any of Claims 1 to 14, preferably in combination with a pharmaceutically acceptable excipient.

18. A process for the preparation of a compound of Formula I

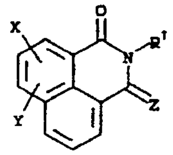

I

in which

Z is an oxo group or two hydrogens;

X and Y are independently selected from hydrogen, halogen, hydroxy, lower alkoxy, benzyloxy, lower alkyl, lower cycloalkyl, nitro, amino, amino-carbonyl, (lower alkyl)amino, di(lower alkyl)amino and (lower alkanoyl)amino;

$R^1$ is selected from

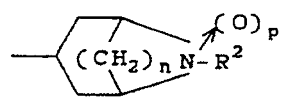

(a)

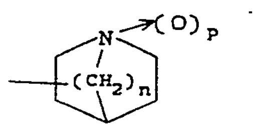

(b)

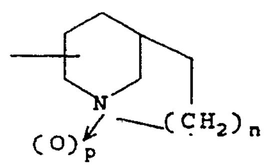

(c)

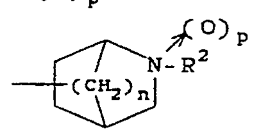

(d)

in which

p is 0 or 1;

n is 1, 2 or 3; and

$R^2$ is hydrogen, lower alkyl, $C_{3-8}$ cycloalkyl, $C_{3-8}$ cycloalkyl-$C_{1-2}$ alkyl, or a group $(CH_2)_tR^3$ where t is 1 or 2 and $R^3$ is thienyl, pyrrolyl or furyl optionally further substituted by one or two substituents selected from lower alkyl, lower alkoxy, trifluoromethyl or halogen, or is phenyl optionally substituted by one or two substituents selected from $C_{1-4}$ alkoxy, trifluoromethyl, halogen, nitro, carboxy, esterified carboxy, and $C_{1-4}$ alkyl optionally substituted by hydroxy, $C_{1-4}$ alkoxy, carboxy, esterified carboxy or in vivo hydrolyzable acyloxy; or

a pharmaceutically acceptable salt thereof, which process comprises one or more of the following steps:

a) the condensation of a 1,8-naphthalic anhydride of the Formula II below with an amine of the formula $R^1NH_2$ wherein $R^1$ has the above meanings or the intramolecular condensation of a compound of the Formula III (IIIA or IIIB) below;

b) the reduction of an isoquinoline-1,3-dione of the Formula IA below to an isoquinoline-3-one of the Formula IB below or the oxidation of a compound of the Formula IB to a compound of the Formula IA;

c) the introduction or removal of a substituent X or Y in a compound of the Formula I to produce another compound of the Formula I;

d) the conversion of one substituent X or Y into another substituent X or Y;

e) the oxidation of a compound of the Formula I wherein p is zero to the corresponding N-oxide of the compound of the Formula I;
f) the reduction of an N-oxide of the compound of the Formula I to the corresponding compound of the Formula I wherein p is zero;
g) the aromatization of the ring defined by carbon atoms 6a, 7, 8, 9, 9a and 10 to afford a compound of the Formula I;
h) the conversion of a salt of a compound of the Formula I to the corresponding free compound or of a free compound of the Formula I to the corresponding salt;
i) the conversion of a salt of a compound of the Formula I to another salt of the compound of the Formula I;
j) the separation of a mixture of isomers or diastereomers derived from a compound of Formula I into a single isomer or diastereomer; or
k) the stereoselective performance of any of the steps a) through i) with optically active reactants.

**19.** A process according to Claim 18 wherein
(1) the condensation step a) comprises reactively contacting a compound of Formula II

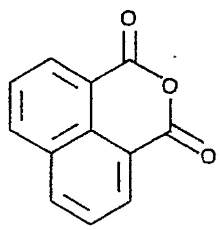

II

with an appropriate amine until a product with the structure of Formula I is formed wherein Z is oxygen;
(2) the reduction step b) comprises reducing with a boranate one of the carbonyl groups in an isoquinoline-1,3-dione of the Formula I to form a compound wherein Z in Formula I represents two hydrogens;
(3) the substituent conversion step d) comprises reducing a nitro substituent in Formula I to form a compound of Formula I wherein the corresponding substituent is amino;
(4) the substituent introduction c) comprises introducing substituents onto an aromatic ring of Formula I activated by a X substituent such as $NH_2$ to form a compound of Formula I wherein Y is $NH_2$ and Cl, Br, I or $NO_2$;
(5) the substituent conversion step d) comprises acylating an amino substituent in Formula I to form a compound of Formula I wherein the corresponding substituent is acetamido;
(6) the substituent conversion step d) comprises substituting nitro or halo substituents in Formula I to form a compound of Formula I wherein the corresponding substituent is lower alkoxy, lower alkylamino or lower dialkylamino;
(7) the salt conversion step h) comprises treating a free base of a compound of Formula I with a pharmaceutically acceptable acid to form the corresponding pharmaceutically acceptable salt;
(8) the oxidation step e) comprises oxidizing a compound of Formula I with a peracid to form the corresponding N-oxide of the $R^1$ component of Formula I; or
(9) the stereoselective step k) comprises the use of (S)-$R^1NH_2$ wherein $R^1$ has the above meanings as the optically active reagent.

**20.** A process according to Claim 18 or 19 wherein $R^1$ is selected from:
1-azabicyclo[2.2.2]oct-3-yl;
1-azabicyclo[2.2.2]oct-4-yl;
endo-9-methyl-9-azabicyclo[3.3.1]non-3-yl;
exo-9-methyl-9-azabicyclo[3.3.1]non-3-yl;
endo-8-methyl-8-azabicyclo[3.2.1]oct-3-yl;
exo-8-methyl-8-azabicyclo[3.2.1]oct-3-yl;

endo-1-azabicyclo[3.3.1]non-4-yl; or
exo-1-azabicyclo[3.3.1]non-4-yl.

**21.** Compounds represented by the formulas

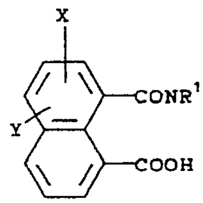

IIIA

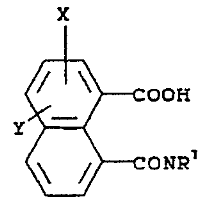

IIIB

wherein

X and Y are independently selected from hydrogen, halogen, hydroxy, lower alkoxy, lower alkyl, lower cycloalkyl, nitro, amino, amino-carbonyl, (lower alkyl)amino, di(lower alkyl)amino and (lower alkanoyl)amino;

$R^1$ is selected from

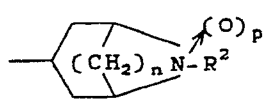

(a)

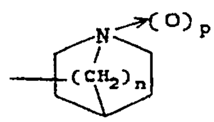

(b)

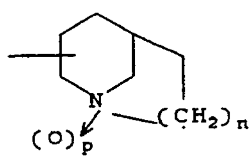

(c)

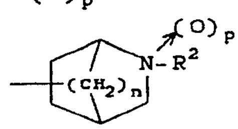

(d)

in which

p is 0 or 1;

n is 1, 2 or 3; and

$R^2$ is hydrogen, lower alkyl, $C_{3-8}$ cycloalkyl, $C_{3-8}$ cycloalkyl-$C_{1-2}$ alkyl, or a group $(CH_2)_tR^3$ where t is 1 or 2 and $R^3$ is thienyl, pyrrolyl or furyl optionally further substituted by one or two substituents selected from lower alkyl, lower alkoxy, trifluoromethyl or halogen, or is phenyl optionally substituted by one or two substituents selected from $C_{1-4}$ alkoxy, trifluoromethyl, halogen, nitro, carboxy, esterified carboxy, and $C_{1-4}$ alkyl optionally substituted by hydroxy, $C_{1-4}$ alkoxy, carboxy, esterified carboxy or in vivo hydrolyzable acyloxy; or

an individual isomer or mixture of isomers thereof, or salts or reactive derivatives thereof.

**22.** A process for preparing a compound of Formula IIIA or IIIB of Claim 21 which process comprises
(a) reacting a compound of the Formula II

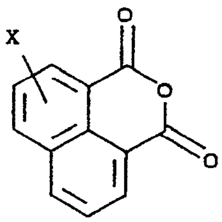

(II)

optionally substituted with Y in the ring which carries the X-substituent, wherein X and Y have the above meanings with a compound of the Formula $R^1NH_2$, or a salt thereof wherein $R^1$ has the above meanings to form the compound of Formula III or its salt;
(b) converting an optionally substituted compound of the Formula II to another optionally substituted compound of the Formula II; or
(c) converting an optionally substituted compound of Formula II to its salt or reactive derivative thereof.

European Patent Office

# EUROPEAN SEARCH REPORT

Application Number

**EP 91 10 7721**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP-A-0 315 390 (BEECHAM GROUP PLC)<br>* page 10, lines 51 - 53; claims 1, 10, 11 * | 1,15-17 | C 07 D 451/04<br>C 07 D 451/14<br>C 07 D 453/02<br>C 07 D 453/06<br>C 07 D 487/08<br>C 07 D 209/80<br>A 61 K 31/40<br>A 61 K 31/435 //<br>(C 07 D 487/08<br>C 07 D<br>C 07 D 209:00<br>C 07 D 209:00 ) |
| A | EP-A-0 093 488 (BEECHAM GROUP PLC)<br>* claims 1, 9, 10 * | 1,15-17 | |
| A,P | EP-A-0 404 737 (ISTITUTO DE ANGELI S.P.A.)<br>* claims 1, 8-11 * | 1,15-17 | |
| A | EP-A-0 247 266 (BEECHAM GROUP PLC)<br>* claims 1, 13-16 * | 1,15-17 | |
| A | EP-A-0 200 444 (BEECHAM GROUP PLC)<br>* claims 1, 15-17 * | 1,15-17 | |
| A,D | BULLETIN OF THE CHEMICAL SOCIETY OF JAPAN, vol. 61, no. 5, 1988, pages 2238 - 2240; R. SATO et al.: "New and Convenient Synthesis of 2-Substituted 2,3-Dihydro-1H-benz[de]isoquinolin-1-ones"<br>* page 2238, scheme 1 * | 18,19 | |
| A,D | TETRAHEDRON LETTERS, vol. 22, no. 19, 1981, pages 2303 - 2306; M. ALEXIOU et al.: "Nucleophilic displacement of the nitro group in 2- and 4-nitronaphthalic-1,8-anhydrides and their derivatives"<br>* page 2304 * | 18,19 | **TECHNICAL FIELDS SEARCHED (Int. Cl.5)**<br>C 07 D 209/00<br>C 07 D 451/00<br>C 07 D 453/00<br>C 07 D 487/00 |

The present search report has been drawn up for all claims

| Place of search | Date of completion of search | Examiner |
|---|---|---|
| Berlin | 23 August 91 | HASS C V F |

CATEGORY OF CITED DOCUMENTS

- X: particularly relevant if taken alone
- Y: particularly relevant if combined with another document of the same catagory
- A: technological background
- O: non-written disclosure
- P: intermediate document
- T: theory or principle underlying the invention
- E: earlier patent document, but published on, or after the filing date
- D: document cited in the application
- L: document cited for other reasons
- &: member of the same patent family, corresponding document